# FASCICULE DE BREVET EUROPEEN

(11) **EP 2 185 558 B1**
(45) Date de publication et mention de la délivrance du brevet: **18.06.2014**
(21) Numéro de dépôt: 08839139.6
(22) Date de dépôt: 31.07.2008
(51) Int. Cl.: C07D 471/04, A61K 31/437, A61P 1/04, A61P 3/00, A61P 13/00, A61P 15/00, A61P 17/00, A61P 11/00, A61P 25/00, A61P 29/00, A61P 31/12, A61P 31/22, C07D 471/14, C07D 233/02, C07D 211/56, A61P 27/14, A61P 1/00

(54) **DÉRIVÉS DE N-HÉTÉROARYL-CARBOXAMIDES TRICYCLIQUES, LEUR PRÉPARATION ET LEUR APPLICATION EN THÉRAPEUTIQUE**
TRICYCLISCHE N-HETEROARYL-CARBOXAMID-DERIVATE SOWIE DEREN HERSTELLUNG UND THERAPEUTISCHE ANWENDUNG
TRICYCLIC N-HETEROARYL-CARBOXAMIDE DERIVATIVES, PREPARATION THEREOF AND THERAPEUTIC USE OF SAME

(30) Priorité: 02.08.2007 FR 0705677
(43) Date de publication de la demande: 19.05.2010
(73) Titulaire: SANOFI, 75008 Paris (FR)
(72) Inventeur: DUBOIS, Laurent, F-75013 Paris (FR); EVANNO, Yannick, F-75013 Paris (FR); MALANDA, André, F-75013 Paris (FR)
(74) Mandataire: Le Coupanec, Pascale A.M.P.
(86) Numéro de dépôt international: PCT/FR2008/001141
(87) Numéro de publication internationale: WO 2009/050348

(56) Documents cités:
- WO-A-2004/072069
- WO-A-2004/108133
- WO-A-2005/016922
- WO-A-2007/010138
- WO-A-2007/010144
- WO-A-2007/088277
- KYLE DONALD J ET AL: "TRPV1 antagonists: a survey of the patent literature" EXPERT OPINION ON THERAPEUTIC PATENTS, ASHLEY PUBLICATIONS, GB, vol. 16, no. 7, juillet 2006 (2006-07), pages 977-996, XP002464449 ISSN: 1354-3776

## Description

Les documents WO2006/024776. WO2006/072736, WO2007/010144 et WO2007/010138 décrivent des dérivés de N-hétéroaryl-carboxamides bicycliques, présentant une activité antagoniste *in vitro* et *in vivo* pour les récepteurs de type TRPV1 (ou VR1).

Il existe toujours un besoin de trouver de nouveaux antagonistes pour les récepteurs de type TRPV1.

La présente invention répond à ce besoin en fournissant des dérivés de *N*-hétéroaryl-carboxamides tricycliques qui présentent une activité antagoniste in *vitro* et in *vivo* pour les récepteurs de type TRPV1 (ou VR1).

Un premier objet de l'invention concerne des composés répondant à la formule générale (I), et définie en détail ci-après.

Un autre objet de l'invention concerne des procédés de préparation de ces composés de formule générale (I).

Un autre objet de l'invention concerne l'utilisation de ces composés de formule générale (I) notamment dans des médicaments ou dans des compositions pharmaceutiques.

La présente invention décrit des composés répondant à la formule générale (I) : 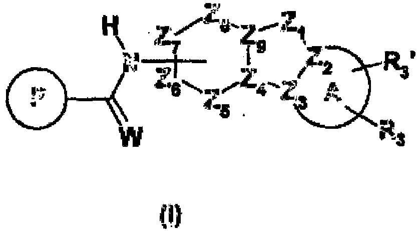 dans laquelle :
A représente, avec la liaison Z₂-Z₃ du bicycle auquel il est fusionné, un groupe C₄-C₇-cycloalkyle, un hétéroaryle monocyclique ou un hétérocycle monocyclique de 4 à 7 chaînons comprenant de un à trois hétéroatomes choisi parmi O, S ou N, y compris les atomes Z₂ et Z₃ ;
Z₁, Z₅, Z₆, Z₇ et Z₈ représentent, indépendamment l'un de l'autre, un atome d'azote, un atome de carbone ou un groupe C-R₂ ;
Z₂, Z₃, Z₄ et Z₉ représentent, indépendamment l'un de l'autre, un atome d'azote ou un atome de carbone et Z₁, Z₂, Z₃, Z₄, Z₅, Z₆, Z₇, Z₈ et Z₉ forment ensemble un hétéroaryle bicyclique lié à l'atome d'azote de l'amide de formule (I) par les positions Z₅, Z₆, Z₇ ou Z₈ quand ces positions correspondent à un atome de carbone, l'un au moins des Z₄, Z₅, Z₆, Z₇, Z₈ et Z₉ correspondant à un atome d'azote ;
W représente un atome d'oxygène ou de soufre ;
P représente un groupe indolyle, pyrrolo[2,3-c]pyridinyle, pyrrolo[2,3-*b*]pyridinyle, pyrrolo[3,2-*b*]pyridinyle ou pyrrolo[3,2-c]pyridinyle tel que représenté ci dessous, lié en position C-2 au carbonyle de l'amide de formule (I) : 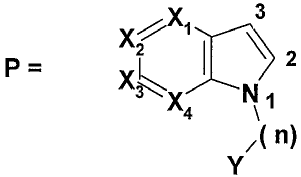 X₁, X₂, X₃, X₄ représentent, indépendamment l'un de l'autre, un atome d'azote ou un groupe C-R₁ ; quand l'un des X_{1,} X₂, X₃ ou X₄ représente un atome d'azote, alors les trois autres représentent un groupe C-R₁ ;
R₁ est choisi parmi un atome d'hydrogène, un atome d'halogène, C₁-C₆-alkyle, C₃-C₇-cycloalkyle, C₃-C₇-cycloalkyl-C₁-C₃-alkylène, C₁-C₆-fluoroalkyle, aryloxy-C₁-C₆-alkyle, hétéroaryloxy-C₁-C₆-alkyle, aryl-C₁-C₃-alkylènoxy-C₁-C₆-alkyle, hétéroaryl-C₁-C₃-alkylènoxy-C₁-C₆-alkyle, arylthio-C₁-C₆-alkyle, hétéroarylthio-C₁-C₆-alkyle, aryl-C₁-C₃-alkylène-thio-C₁-C₆-alkyle, hétéroaryl-C₁-C₃-alkylène-thio-C₁-C₆-alkyle, C₁-C₆-alcoxy, C₃-C₇-cycloalkyloxy, C₃-C₇-cycloalkyl-C₁-C₃-alkylènoxy, C₁-C₆-fluoroalcoxy, cyano, C(O)NR₄R₅, nitro, NR₄R₅, C₁-C₆-thioalkyle, C₃-C₇-cycloalkylthio, C₃-C₇-cycloalkyl-C₁-C₃-alkylène-thio, -S(O)-C₁-C₆-alkyle, -S(O)-C₃-C₇-cycloalkyle, -S(O)-C₁-C₃-alkylène-C₃-C₇-cycloalkyle, C₁-C₆-alkyle-S(O)₂-, C₁-C₆-fluoroalkyle-S(O)₂-, C₃-C₇-cycloalkyl-S(O)₂-, C₃-C₇-cycloalkyl-C₁-C₃-alkylène-S(O)₂-, SO₂NR₄R₅, SF₅, NR₆C(O)R₇, NR₆SO₂R₈, C(O)NR₄R₅, OC(O)NR₄R₅, aryle, hétéroaryle, aryl-C₁-C₅-alkylène, hétéroaryl-C₁-C₅-alkylène, aryloxy, arylthio, hétéroaryloxy ou hétéroarylthio, les groupes hétéroaryle ou aryle étant éventuellement substitués par un ou plusieurs substituants R₉, identiques ou différents l'un de l'autre ;
n est égal à 0, 1, 2 ou 3 ;
Y représente un aryle ou un hétéroaryle éventuellement substitué par un ou plusieurs groupes choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, hydroxy, C₁-C₆-alcoxy, C₃-C₇-cycloalkyloxy, C₃-C₇-cycloalkyl-C₁-C₆-alkylène-O-, C₁-C₆-fluoroalcoxy, cyano, C(O) NR₄R₅, nitro, NR₄R₅, C₁-C₆-thioalkyle, thiol, -S(O)-C₁-C₆-alkyle, -S(O)₂-C₁-C₆-alkyle, SO₂NR₄R₅, NR₆C(O)R₇, NR₆SO₂R₈, C(O)NR₄R₅, OC(O)NR₄R₅, aryle-C₁-C₅-alkylène ou aryle, hétéroaryl-C₁-C₅-alkylène ou hétéroaryle, les groupes aryle et hétéroaryle étant éventuellement substitués par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
R₂ représente un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylènoxy, thiol, -S(O)-C₁-C₆-alkyle, -S(O)₂-C₁-C₆-alkyle, hydroxy, C₁-C₆-fluoroalcoxy ;
R₃ et R₃' représentent les substituants de A à l'exclusion des substituants des atomes Z₂ et Z₃ ;
R₃ et R₃', représentent, indépendamment l'un de l'autre, lorsqu'ils sont portés par un atome de carbone, un atome d'hydrogène, un atome d'halogène, un groupe C₁-C₆-alkyle, C₃-C₇-cycloalkyle, C₃-C₇-cycloalkyl-C₁-C₃-alkylène, C₁-C₆-fluoroalkyle, hydroxyle, thiol, C₁-C₆-alcoxy, C₃-C₇-cycloalkyloxy, C₃-C₇-cycloalkyl-C₁-C₃-alkylénoxy, aryle-C(O)-O-, C₁-C₆-alkyle-C(O)-O-, C₃-C₇-cycloalkyl-C(O)-O-, C₃-C₇-cycloalkyl-C₁-C₃-alkylène-C(O)-O, C₁-C₆-fluoroalkyle-C(O)-O-, oxo, thio, NR₄R₅, NR₆C(O)R₇, hétéroaryle ou aryle ; les groupes hétéroaryle et aryle étant éventuellement substitués par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou bien R₃ et R₃' forment ensemble, avec l'atome da carbone qui les porte, un groupe C₃-C₇-cycloalk-1,1-diyle ; ou
R₃ et R₃', représentent indépendamment l'un de l'autre, lorsqu'ils sont portés par un atome d'azote, un atome d'hydrogène, un groupe C₁-C₆-alkyle, C₃-C₇-cycloalkyle, C₃-C₇-cycloalkyl-C₁-C₃-alkylène, C₁-C₅-fluoroalkyle, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆₋fluoroalkyle-C(O)-, aryle-S(O)-, C₁-C₆-alkyle-S(O)-, C₁-C₆-fluoroalkyle-S(O)-, C₃-C₇-cycloalkyl-S(O)-, C₃-C₇-cycloalkyl-C₁-C₃-alkylène-S(O)-, aryle-S(O)₂-, C₁-C₆-alkyle-S(O)₂₋, C₁-C₆-fluoroalkyle-S(O)₂₋, C₃-C₇-cycloalkyl-S(O)₂-, C₃-C₇-cycloalkyl-C₁-C₃-alkylène-S(O)₂-, C₁-C₆-alkyle-O-C(O)-, aryle-C₁-C₃-alkyle-O-C(O)- C₃-C₇-cycloalkyl-OC(O)-, C₃-C₇-cycloalkyl-C₁-C₃-alkylène-O-C(O)-, C₁-C₆-fluoroalkyle-O-C(O)-, aryle-OC(O)-, hétéroaryl-O-C(O)-, hétéroaryle ou aryle ; les groupes hétéroaryle et aryle étant éventuellement substitués par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
R₄ et R₅, représentent, indépendamment l'un de l'autre, un atome d'hydrogène ou un groupe C₁-C₆-alkyle, C₃-C₇-cycloalkyle, C₃-C₇-cycloalkyl-C₁-C₃-alkylène, aryle-C₁-C₆-alkylène ou aryle, ou R₄ et R₅ forment ensemble, avec l'atome d'azote qui les porte, un groupe azétidine, pyrrolidine, pipéridine, azépine, morpholine, thiomorpholine, piperazine, homopipérazine, le groupe NR₄R₅ étant éventuellement substitué par un groupe C₁-C₆-alkyle, C₃-C₇-cycloalkyle, C₃-C₇-cycloalkyl-C₁-C₃-alkylène, aryle-C₁-C₆-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂-, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, hydroxy, C₁-C₆-alkyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
R₆ et R₇ représentent, indépendamment l'un de l'autre, un atome d'hydrogène, un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou R₆ et R₇ forment ensemble un lactame de 4 à 7 chaînons comprenant l'atome d'azote et le groupe C(O) qui les portent ;
R₆ représente un groupe C₁-C₆-alkyle, C₃-C₇-cycloalkyle, C₃-C₇-cycloalkyl-C₁-C₃-alkylène, aryle-C₁-C₆-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-, alkylénoxy, C₁-C₆-fluoroalcoxy, nitro ou cyano ;
ou R₆ et R₈ forment ensemble un sultame de 4 à 7 chaînons comprenant l'atome d'azote et le groupe S(O)₂ qui les portent ;
R₉ représente un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro, cyano, NR₄R₆, R₄R₅N-C₁-C₃-alkylène.

Dans les composés de formule générale (I) :
- le ou les atomes de soufre de l'hétérocycle A peuvent être sous forme oxydée (S(O) ou S(O)₂) ;
- le ou les atomes d'azote peuvent éventuellement être sous forme oxydée (N-oxyde).

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, sont décrit dans l'invention.

Les composés de formule (I) peuvent exister à l'état de bases ou de sels d'addition à des acides. De tels sels d'addition sont décrits dans l'invention.

Ces sels peuvent être préparés avec des acides pharmaceutiquement acceptables, mais les sels d'autres acides utiles, par exemple, pour la purification ou l'isolement des composés de formule (I) sont également décrits dans l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un atome d'halogène : un fluor, un chlore, un brome ou un iode ;
- Cₜ-C_{z} : une chaîne carbonée pouvant avoir de t à z atomes de carbone où t et z peuvent prendre les valeurs de 1 à 7 ; par exemple C₁-C₃ est une chatne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- un cycloalkyle : un groupe alkyle cyclique, saturé ou partiellement insaturé. A titre d'exemples, on peut citer les groupes cyclopropyle, cyclobutyle, cyclopentyle, cyclohexyle, etc ;
- un cycloalk-1,1-diyle : un groupe du type 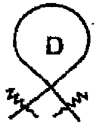 dans lequel D représente un groupe cycloalkyle ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un thioalkyle ou alkyle-thio : un radical -S-alkyle où le groupe alkyle est tel que précédemment défini ;
- un aryle: un groupe aromatique mono- ou bicyclique comprenant entre 6 et 10 atomes de carbones. A titre d'exemples de groupe aryle, on peut citer les groupes phényle ou naphthyle ;
- un hétérocycle : un groupe monocyclique saturé ou partiellement insaturé, de 4 à 8 chaînons, comprenant de 1 à 5 hétéroatomes choisis parmi O, S ou N.

A titre d'exemples d'hétérocycle, on peut citer les groupes azétidinyle, pyrrolidinyle, pipéridinyle, azépinyle, morpholinyle, thiomorpholinyle, pipérazinyle, homopipérazinyle, dihydrooxazolyle, dihydrothiazolyle, dihydroimidazolyle, dihydropyrrolyle ou tétrahydropyridinyle, tétrahydro[1,2]oxazine tétrahydro[1,3]oxazine, tetrahydro[1,4]oxazine, [1,3]oxazépine ou [1,4]oxazépine.
- un hétéroaryle : un groupe mono- ou bicyclique aromatique de 5 à 12 chaînons contenant de 1 à 5 hétéroatomes choisis parmi O, S ou N.

A titre d'exemples d'hétéroaryle monocyclique, on peut citer les groupes imidazolyle, pyrazolyle, thiazolyle, oxazolyle, isothiazolyle, isoxazolyle, furanyle, thiophényle, oxadiazolyle, thiadiazolyle, triazolyle, tétrazolyle, pyridinyle, pyrazinyle, pyrimidinyle, pyridazinyle, triazinyle.

A titre d'exemples d'hétéroaryle bicyclique, on peut citer les groupes indolyle, isoindolyle, benzofuranyle, benzothiophényle, benzoxazolyle, benzimidazolyle, indazolyle, benzothiazolyle, isobenzofuranyle, isobenzothiazolyle, pyrrolo[2,3-c]pyridinyle, pyrrolo[2,3-*b*]pyridinyle, pyrrolo[3,2-*b*]pyridinyle, pyrrolo[3,2-*c*]pyridinyle, pyrrolo[1,2-*a*]pyridinyle, quinoléinyle, isoquinoléinyle, cinnolinyle, quinazolinyle, quinoxalinyle, pyrrolo[1,2-*a*]imidazolyle, imidazo[1,2-*a*]pyridinyle, imidazo[1,2-*a*]pyridazinyle, imidazo[1,2*-c*]pyrimidinyle, imidazo[1,2-*a*]pyrimidinyle, imidazo[1,2-*a*]pyrazinyle, imidazo[4,5-*b*]pyrazinyle, imidazo[4,5-*b*]pyridinyle, imidazo[4,5-*c*]pyridinyle, pyrazolo[2,3-a]pyridinyle, pyrazolo[2,3-*a*]pyrimidinyle, pyrazolo[2,3-*a*]pyrazinyle.
- « oxo » signifie « =O » ;
- « thio » signifie « =S ».

Parmi les composés de formule générale (I) décrits dans l'invention, un premier groupe de composés est constitué par les composés qui répondent à la formule générale (Ia) :

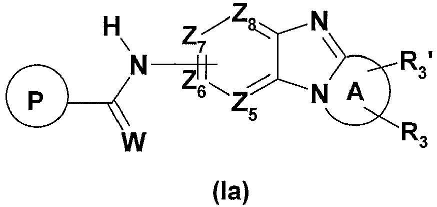

A, Z₅, Z₆, Z₇, Z₈, P, W, R₃ et R₃' étant tels que définis dans la formule générale (I).

Parmi les composés de formule générale (I) décrits dans l'invention, un second groupe de composés est constitué par les composés qui répondent à la formule générale (Ib) :

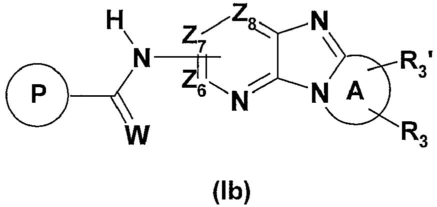

Z₆, Z₇ et Z₈ représentent, indépendamment l'un de l'autre, un atome de carbone ou un groupe C-R₂ ; l'un des Z₆, Z₇ ou Z₈ correspond à un atome de carbone et est lié à l'atome d'azote de l'amide de formule (Ib) ;
A, P, W, R₂, R₃ et R₃' étant tels que définis dans la formule générale (I).

Parmi les composés de formule générale (I) décrits dans l'invention, un troisième groupe de composés est constitué par les composés de formule générale (I) pour lesquels le groupe 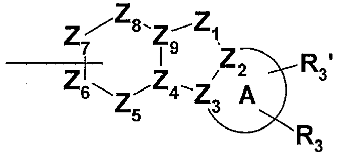 est choisi parmi : 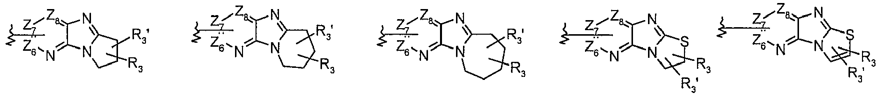 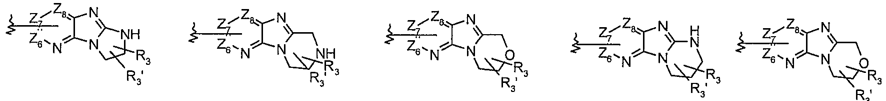 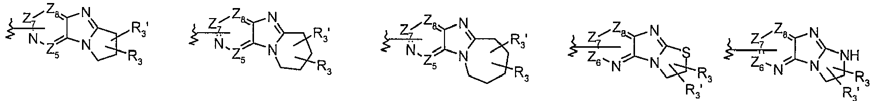 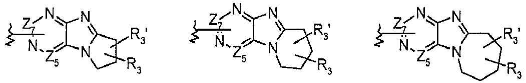 Z₅, Z₆, Z₇ et Z₈ représentent, indépendamment l'un de l'autre, un atome de carbone lié à l'atome d'azote de l'amide de formule (I) ou un groupe C-R₂ ;
R₂, R₃ et R₃' étant tels que définis dans la formule générale (I).

Parmi les composés de formule générale (I) décrits dans l'invention, un quatrième groupe de composés est constitué par les composés de formule générale (I) pour lesquels
R₂ représente un atome d'hydrogène ;
R₃ et R₃' sont portés par un atome de carbone et représentent, indépendamment l'un de l'autre, un atome d'hydrogène, un atome d'halogène, un groupe C₁-C₆-alkyle, hydroxyle, C₁-C₆-alkyle-C(O)-O-, NR₄R₅, NR₆C(O)R₇ ou aryle ;
R₄ et R₅, représentent, indépendamment l'un de l'autre, un atome d'hydrogène ou un groupe C₁-C₆-alkyle ;
R₆ et R₇ représentent, indépendamment l'un de l'autre, un atome d'hydrogène ou un groupe C₁-C₆-alkyle.

Parmi les composés de formule générale (I) décrits dans l'invention, un cinquième groupe de composés est constitué par les composés de formule générale (I) pour lesquels
le groupe 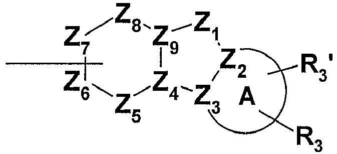 est choisi parmi : 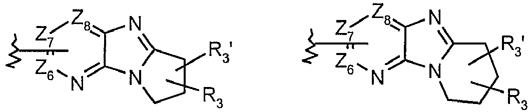 Z₆, Z₇ et Z₈ représentent, indépendamment l'un de l'autre, un atome de carbone lié à l'atome d'azote de l'amide de formule (I) ou un groupe C-R₂ ; R₂ représente un atome d'hydrogène ;
R₃ et R₃' sont portés par un atome de carbone et représentent, indépendamment l'un de l'autre, un atome d'hydrogène, un atome d'halogène, un groupe C₁-C₆-alkyle, hydroxyle, C₁-C₆-alkyle-C(O)-O-, NR₄R₅, NR₆C(O)R₇ ou aryle ;
R₄ et R₅, représentent, indépendamment l'un de l'autre, un atome d'hydrogène ou un groupe C₁-C₆-alkyle ;
R₆ et R₇ représentent, indépendamment l'un de l'autre, un atome d'hydrogène ou un groupe C₁-C₆-alkyle.

Parmi les composés de formule générale (I) décrits dans l'invention, un sixième groupe de composés est constitué par les composés de formule générale (I) pour lesquels W représente un atome d'oxygène.

Parmi les composés de formule générale (I) décrits dans l'invention, un septième groupe de composés est constitué par les composés de formule générale (I) pour lesquels
P représente un groupe indolyle ou pyrrolo[2,3-*b*]pyridinyle tel que représenté ci dessous, lié en position C-2 au carbonyle de l'amide de formule (I) : 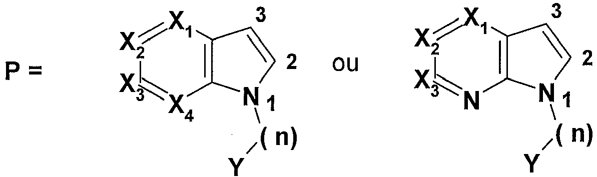 X₁, X₂, X₃, X₄ représentent, indépendamment l'un de l'autre, un groupe C-R₁ ; R₁, Y et n étant tels que définis dans la formule générale (I).

Parmi les composés de formule générale (I) décrits dans l'invention, un huitième groupe de composés est constitué par les composés de formule générale (I) pour lesquels
R₁ est choisi parmi un atome d'hydrogène, un atome d'halogène, C₁-C₆-alkyle, C₁-C₆-fluoroalkyle, C₁-C₆-thioalkyle, C₁-C₆-alkyle-S(O)₂-.

Parmi les composés de formule générale (I) décrits dans l'invention, un neuvième groupe de composés est constitué par les composés de formule générale (I) pour lesquels n est égal à 1.

Parmi les composés de formule générale (I) décrits dans l'invention, un dixième groupe de composés est constitué par les composés de formule générale (I) pour lesquels Y représente un aryle, plus particulièrement un phényle, éventuellement substitué par un ou plusieurs atomes d'halogène, plus particulièrement de fluor.

Parmi les composés de formule générale (I) décrits dans l'invention, un onzième groupe de composés est constitué par les composés de formule générale (I) pour lesquels
P représente un groupe indolyle ou pyrrolo[2,3-*b*]pyridinyle tel que représenté ci dessous, lié en position C-2 au carbonyle de l'amide de formule (I) : 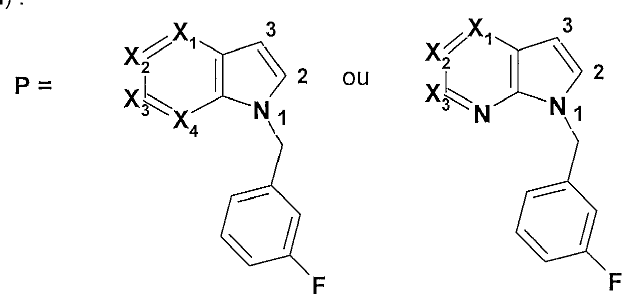 X₁, X₂, X₃, X₄ représentent, indépendamment l'un de l'autre, un groupe C-R₁ ;
R₁ est choisi parmi un atome d'hydrogène, un atome d'halogène, C₁-C₆-alkyle, C₁-C₆-fluoroalkyle, C₁-C₆-thioalkyle, C₁-C₆-alkyle-S(O)₂-.

Ainsi, la présente invention concerne les composés de formule générale (I) 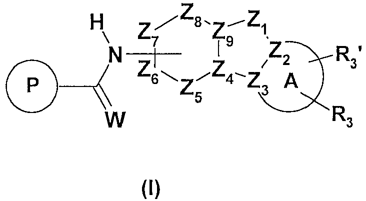 dans laquelle le groupe: 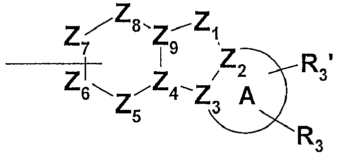 est choisi parmi : 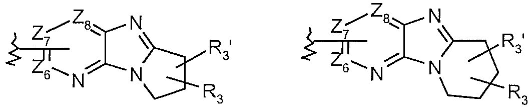 Z₆, Z₇ et Z₈ représentent, indépendamment l'un de l'autre, un atome de carbone lié à l'atome d'azote de l'amide de formule (I) ou un groupe C-R₂ ;
R₂ représente un atome d'hydrogène ;
R₃ et R₃' sont portés par un atome de carbone et représentent, indépendamment l'un de l'autre, un atome d'hydrogène, un atome d'halogène, un groupe C₁-C₆-alkyle, hydroxyle, C₁-C₆-alkyle-C(O)-O-, NR₄R₅, NR₆C(O)R₇ ou aryle ;
R₄ et R₅, représentent, indépendamment l'un de l'autre, un atome d'hydrogène ou un groupe C₁-C₆-alkyle ;
R₆ et R₇ représentent, indépendamment l'un de l'autre, un atome d'hydrogène ou un groupe C₁-C₆-alkyle.
W représente un atome d'oxygène ;
P représente un groupe indolyle ou pyrrolo[2,3-*b*]pyridinyle tel que représenté ci dessous, lié en position C-2 au carbonyle de l'amide de formule (I) : 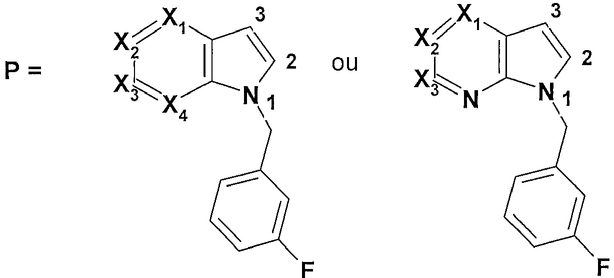 X₁, X₂, X₃, X₄ représentent, indépendamment l'un de l'autre, un groupe C-R₁ ;
R₁ est choisi parmi un atome d'hydrogène, un atome d'halogène, C₁-C₆-alkyle, C₁-C₆-fluoroalkyle, C₁-C₆-thioalkyle, C₁-C₆-alkyle-S(O)₂- ;
le ou les atomes d'azote pouvant éventuellement être sous forme oxydée (N-oxyde) à l'état de base ou de sel d'addition à un acide, ainsi qu'à l'état hydrate ou de solvate.

Parmi les composés de formule générale (I) décrits dans l'invention, un douzième groupe de composés est constitué par les composés de formule générale (I) dans laquelle à la fois P et/ou W et/ou Z₁ et/ou Z₂ et/ou Z₃ et/ou Z₄ et/ou Z₅ et/ou Z₆ et/ou Z₇ et/ou Z₈ et/ou Z₉ et/ou A et/ou R₃ et/ou R₃' sont tels que définis dans les groupes définis ci-dessus.

Parmi les composés de formule générale (I) objets de l'invention, on peut notamment citer les composés suivants :

Parmi les composés de formule générale (I) objets de l'invention, on peut notamment citer les composés suivants :
∘ *N*-(7,8-Dihydro-6*H*-pyrrolo[2',1':2,3]imidazo[4,5,*b*]pyridin-3-yl)-5-fluoro-1-(3-fluorobenzyl)-1*H*-indole-2-carboxamide
∘ *N*-(6-Acétoxy-7,8-dihydro-6*H*-pyrrolo[2',1':2,3]imidazo[4,5-*b*]pyridin-3-yl)-5-fluoro-1-(3-fluorobenzyl)-1*H*-indole-2-carboxamide
∘ *N*-(6-Hydroxy-7,8-dihydro-6*H*-pyrrolo[2',1':2,3]imidazo[4,5-*b*]pyridin-3-yl)-5-fluoro-1-(3-fluorobenzyl)-1*H*-indole-2-carboxamide
∘ *N*-(7,8-Dihydro-6*H*-pyrrolo[2',1':2,3]imidazo[4,5-*b*]pyridin-3yl)-4-fluoro-1-(3-fluorobenzyl)-1*H*-indole-2-carboxamide
∘ *N*-(7,8-Dihydro-6*H*-pyrrolo[2',1':2,3]imidazo[4',5-*b*]pyridin-3-yl)-7-fluoro-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-fluoro-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-trifluorométhyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3yl)-5-trifluorométhyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-4-chloro-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-chloro-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-5-chloro-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-1-(3-fluorobenzyl)-1*H*-indole-2-carboxamide
∘ *N*-(7,8-Dihydro-6*H*-pyrrolo[2'1':2,3]imidazo[4,5-*b*]pyridin-3-yl)-4-méthyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-méthyl-1-(3-fluorobenzyl)-1*H*-indole-2-carboxamide
∘ *N*-(7,8-Dihydro-3*H*-pyrrolo[2',1':2,3]imidazo[4,5-*b*]pyridin-3-yl)-5-méthyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-éthyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-isopropyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-5-*tert*butyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-*tert*butyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-4,7-diméthyl-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-5-méthylsulfonyl-1-(3 fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-méthylthio-1-(3-fluorobenzyl)-1*H*-indole-2-carboxamide
∘ *N*-(7,8-Dihydro-6*H*-pyrrolo[2',1':2,3]imidazo[4,5-*b*]pyridin-3-yl)-6-trifluorométhyl-1-(3-fluorobenzyl)-1*H*-pyrrolo[2,3-*b*]pyridine-2-carboxamide
∘ *N*-(7,8-Dihydro-6*H*-pyrrolo[2',1':2,3]imidazo[4,5-*b*]pyridin-3-yl)-5-trifluorométhyl-1-(3-fluorobenzyl)-1*H*-pyrrolo[2,3-*b*]pyridine-2-carboxamide
∘ *N*-(7,8-Dihydro-6*H*-pyrrolo[2',1':2,3]imidazo[4,5-*b*]pyridin-3-yl)-5-fluoro-1-(3-fluorobenzyl)-1*H*-pyrrolo[2,3*-b*]pyridine-2-carboxamide
∘ *N*-(6-Hydroxy-7,8-dihydro-6*H*-pyrrolo[2',1':2,3]imidazo[4,5-*b*]pyridin-3-yl)-5-trifluorométhyl-1-(3-fluorobenzyl)-1*H*-indole-2-carboxamide
∘ *N*-(6,7,8,9-tétrahydri-Imidazo[1,2-*a*:5,4-*b'*]dipyridin-3-yl)-5-trifluorométhyl-1-(3-fluorobenzyl)-1*H*-indole-2-carboxamide
∘ (-)-*N*-(6-Hydroxy-7,8-dihydro-6*H*-pyrrolo[2',1':2,3]imidazo[4,5-*b*]pyridin-3-yl)-6-trifluorométhyl-1-(3-fluorobenzyl-1*H*-indole-2-carboxamide
∘ *N*-(6-Phényl-7,8-dihydro-6*H*-pyrrolo[2',1':2,3]imidazo[4,5-*b*]pyridin-3-yl)-5-trifluorométhyl-1-(3-fluorobenzyl)-1*H*-indole-2-carboxamide
∘ *N*-(6,6-Diméthyl-7,8-dihydro-6*H*-pyrrolo[2',1':2,3]imidazo[4,5-*b*]pyridin-3-yl)-5-trifluorométhyl-1-(3-fluorobenzyl)-1*H*-indole-2-carboxamide
∘ (-)-*N*-(6-Diméthylamino-7,8-dihydro-6*H*-pyrrolo[2',1':2,3]imidazo[4,5-*b*]pyridin-3-yl)-5-trifluorométhyl-1-(3-fluorobenzyl)-1*H*-indole-2-carboxamide
∘ *N*-(6-amino-7,8-dihydro-6*H*-pyrrolo[2',1':2,3]imidazo[4,5-*b*]pyridin-3-yl)-5-fluoro-1-(3-fluorobenzyl)-1*H*-indole-2-carboxamide
∘ *N-*(6-Acétylamino-7,8-dihydro-6*H*-pyrrolo[2',1':2,3]imidazo[4,5-*b*]pyridin-3-yl)-5-fluoro-1-(3-fluorobenzyl)-1*H*-indole-2-carboxamide
∘ (-)-*N*-(6-Hydroxy-7,8-dihydro-6*H*-pyrrolo(2',1':2,3]imidazo[4,5-*b*]pyridin-3-yl)-5-trifluorométhyl-1-(3-fluorobenzyl)-1*H*-pyrrolo[2,3-*b*]pyridine-2-carboxamide
∘ (+)-*N*-(6-Fluoro-7,8-dihydro-6*H*-pyrrolo[2',1':2,3]imidazo[4,5-*b*]pyridin-3-yl)-6-trifluorométhyl-1-(3-fluorobenzyl)-1*H*-indole-2-carboxamide
∘ *N*-(6-Amino-7,8-dihydro-6*H*-pyrrolo[2',1':2,3]imidazo[4,5-*b*]pyridin-3-yl)-5-trifluorométhyl-1-(3-fluorobenzyl)-1*H*-indole-2-carboxamide

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

On entend par groupe protecteur, dans ce qui suit, un groups pouvant être momentanément incorporé à une structure chimique dans le but d'inactiver temporairement une partie de la molécule lors d'une réaction et qui peut être facilement être enlevé à une étape ultérieure de la synthèse. Des exemples de groupes protecteurs ainsi que des références concernant leurs propriétés sont donnés dans T.W. Greene, P.G.M. Wutz, 3rd Edition, Wiley interscience 1999.

On peut préparer les composés de formule générale (I) selon le procédé illustré par le schéma général 1 qui suit :

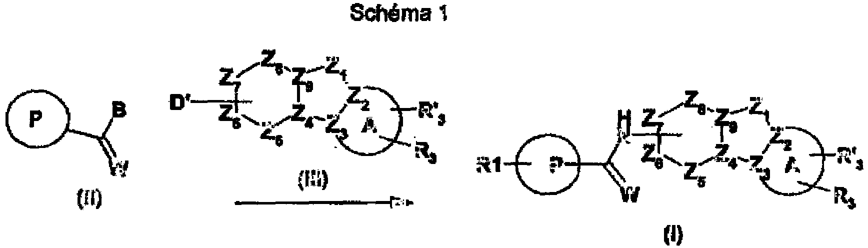

Les composés (I) peuvent être obtenus par réaction d'un composé de formule générale (II) dans laquelle B représente un groupe C₁-C₆-alcoxy, C₃C₇-cycloalkyloxy, C₃-C₇-cycloalkyl-C₁-C₃-alkylénoxy, aryle-C₁-C₃-alkylénoxy, et W et P sont tels que définis dans la formule générale (I), avec un composé de formule générale (III), dans laquelle D' représente un groupe amidure et A, Z₁, Z₂, Z₃, Z₄, Z₅, Z₆, Z₇, Z₈, Z₉, R₃ et R₃' sont tels que définis dans la formule générale (I) ci-dessus, ou reflux d'un solvant tel que le toluène. L'amidure d'aluminium du composé de formule générale (III) peut être préparé par action préalable du triméthylaluminium sur les amines de formule générale (III) dans laquelle D' représente un groupe NH₂.

En partant de composés de formule générale (II), dans laquelle B représente un groupe hydroxyle, W représente un atome d'oxygène, et P est tel que défini dans la formule générale (I), la fonction acide carboxylique peut préalablement être transformée en halogénure d'acide tel qu'un chlorure d'acide par action, par exemple, du chlorure de thionyle, au reflux d'un solvant tel que la dichlorométhane ou le dichloroéthane. Le composé de formule générale (I) peut alors obtenu par réaction des composés de formule générale (II), dans laquelle B représente un atome de chlore, W représente un atome d'oxygène, et P est tel que défini dans la formule générale (I), avec le composé de formule générale (III), dans laquelle A, Z₁, Z₂, Z₃, Z₄, Z₅, Z₆, Z₇, Z₈, Z₉, R₃ et R₃' sont tels que définis dans la formule générale (I) ci-dessus et D' représente un groupe NH₂, en présence d'une base telle que la triéthylamine ou le carbonate de sodium.

Alternativement, les composés de formule générale (II), dans laquelle B représente un groupe hydroxyle, W représente un atome d'oxygène, et P est tel que défini dans la formule générale (I), peuvent être couplés avec les composés de formule générale (III), dans laquelle A, Z₁, Z₂, Z₃, Z₄, Z₅, Z₆, Z₇, Z₈, Z₉, R₃ et R₃' sont tels que définis dans la formule générale (I) ci-dessus et D' représente un groupe NH₂, en présence d'un agent de couplage tel qu'un dialkylcarbodiimide, l'hexafluorophosphate de [(benzotriazol-1-yl)oxy][tris(pyrrolidino)]phosphonium, le diéthylcyanophosphonate ou tout autre agent de couplage connu de l'homme du métier, en présence d'une base comme la triéthylamine, dans un solvant tel que par exemple le diméthylformamide.

En partant de composés de formule générale (II), dans laquelle B représente un groupe NH₂, W représente un atome d'oxygène, et P est tel que défini dans la formule générale (I), le composé de formule générale (I) peut être obtenu par réaction avec le composé de formule générale (III), dans laquelle A, Z₁, Z₂ Z₃, Z₄, Z₅, Z₆, Z₇, Z₈, Z₉, R₃ et R₃' sont tels que définis dans la formule générale (I) ci-dessus et D' correspond à un groupe partant tel que défini ci-dessus, tel qu'un atome de brome ou un groupe triflate, par exemple selon une méthode analogue à celle décrite dans J.Am.Chem.Soc. 2001, 123 (31), 7727, ou selon des méthodes décrites dans la littérature ou connues de l'homme du métier, en présence d'un sel de cuivre en quantité catalytique, en présence d'une quantité catalytique d'un ligand du cuivre, tel qu'une diamine, le tout en présence d'une base comme le carbonate de potassium, dans un solvant tel que le dioxane.

Dans le schéma 1, les composés de formule générale (II) et les autres réactifs, quand leur mode de préparation n'est pas décrit, sont disponibles dans le commerce, décrits dans la littérature ou préparés par analogie à des procédés décrits dans la littérature (D. Knittel Synthesis 1883, 2, 186 ; T.M. Williams J.Med.Chem. 1993, 36 (9), 1291 ; JP2001151771A2 ; WO2006024776 ; WO2007010138 par exemple).

L'invention vise également un procédé de préparation d'un composé de formule (I) selon l'invention, caractérisé en ce que l'on fait réagir un composé de formule générale (II) : 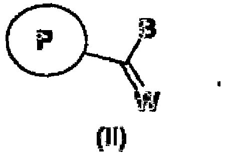 dans laquelle P et W sont tels que définis dans la formule générale (I) selon l'invention,
avec un composé de formule générale (III), 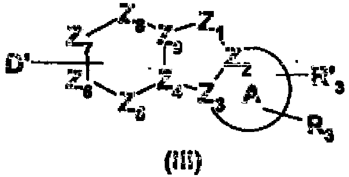 dans laquelle A, Z₁, Z₂, Z₃, Z₄, Z₅, Z₆, Z₇, Z₈, Z₉, R₃ et R₃' sont tels que définis dans la formule générale (I) selon l'invention
- quand B représente un groupe C₁-C₆-alcoxy, C₃-C₇-cycloalkyloxy, C₃-C₇-cycloalkyl-C₁-C₃-alkylénoxy, aryle-C₁-C₃-alkylénoxy, avec un amidure du composé de formule générale (III), au reflux d'un solvant, l'amidure d'aluminium du composé de formule générale (III) étant préparé par action préalable du triméthylaluminium sur le composé de formule générale (III), dans laquelle D' représente un groupe NH₂ ;
- quand B représente un groupe hydroxyle et W représente un atome d'oxygène,
   soit par transformation de la fonction acide carboxylique en halogénure d'acide puis par réaction du composé de formule générale (II), dans laquelle B représente un atome de chlore et W représente un atome d'oxygène, avec le composé de formule générale (III), dans laquelle D' représente un groupe NH₂, en présence d'une base ;
   soit par couplage du composé de formule générale (II) avec les composés de formule générale (III), dans laquelle D' représente un groupe NH₂, en présence d'un agent de couplage et d'une base dans un solvant ;
- quand B représente un groupe NH₂ et W représente un atome d'oxygène, par réaction du composé de formule générale (II) avec le composé de formule générale (III), dans laquelle D' correspond à un groupe partent, en présence d'un sel de cuivre en quantité catalytique, en présence d'une quantité catalytique d'un ligand du cuivre, le tout en présence d'une base dans un solvant.

Les composés de formule générale (II) ou (I), C-substitués sur un groupe aryle ou hétéroaryle par un groupe alkyle, peuvent être obtenus par une réaction de couplage, catalysée par un métal tel que le palladium ou le fer, réalisée sur les composés de formules générales (II) ou (I) correspondants, substitués par un atome d'halogène, tel qu'un chlore, en présence par exemple d'un halogénure d'alkylmangnésium ou d'un halogénure d'alkylzinc, selon les méthodes décrites dans la littérature (A. Fursiner et al J Am Chem Soc 2002, 124(46), 13856 ; G. Quéguiner et al J Org Chem 1998, 63(9), 2892) par exemple, ou connues de l'homme du métier.

Les composés de formule générale (II) ou (I), C-substitués sur un groupe aryle ou hétéroaryle par un groupe cyano, aryle ou hétéroaryle, peuvent être obtenus par une réaction de couplage, catalysée par un métal tel que le palladium, réalisée sur les composés de formule générale (II) ou (I) correspondants, substitués, par exemple, par un atome de brome, en présence de cyanure de triméthylsilyle, d'acide arylboronique ou d'acide hétéroarylarylboronique, ou par toute autre méthode décrite dans la littérature ou connue de l'homme du métier.

Les composés de formule générale (II) ou (I), dans laquelle P est N-substitué par un substituant R₁ correspondant à un groupe aryle ou héteroaryle, peuvent être obtenus par une réaction de couplage, catalysée par un métal tel que le cuivre, réalisés sur les amines de formule générale (II) ou (I) correspondantes, en présence d'un halogénure d'aryle ou d'hétéroaryle, selon la méthode de Buchwald (S.L. Buchwald et al J Am Chem Soc 2002, 124, 11684), ou par toute autre méthode décrite dans la littérature ou connue de l'homme du métier.

Les composés de formule générale (I) ou (II) C-subsbtués sur un groupe aryle ou hétéroaryle par un groupe NR₄R₅, NR₅COR₇ ou NR₈SO₂R₈, peuvent être obtenus à partir des composés de formule générale (I) ou (II) correspondants, substitués, par exemple, par un atome de brome, par réaction de couplage respectivement avec une amine, un amide ou une sulfonamide en présence d'une base, d'une phosphine et d'un catalyseur à base de palladium, selon des méthodes décrites dans la littérature ou connues de l'homme du métier.

Les composés de formule générele (I) ou (II) substitués par un groupe C(O)NR₄R₅, peuvent être obtenue à partir des composés de formule générale (I) ou (II) correspondants substitués par un groupe cyano, selon des méthodes décrites dans la littérature ou connues de l'homme du métier.

Les composés de formule générale (I) ou (II) substitués par un groupe -S(O)-alkyle ou - S(O)₂-alkyle, peuvent être obtenus par oxydation des composés de formule générale (II) ou (I) correspondants, substitués par un groupe thioalkyle, selon des méthodes décrites dans la littérature ou connues de l'homme du métier.

Les composés de formule générale (II) ou (J), substitués par un groupe NR₄R₅, NR₆COR₇ ou NR₆SO₂R₈, peuvent être obtenus à partir des composés de formule générale (II) ou (I) correspondants, substitués par un groupe nitro, par exemple par réduction, puis acylation ou sulfonylation, selon des méthodes décrites dans la littérature ou connues de l'homme du métier.

Les composés de formule générale (II) ou (I), substitués par un groupe SO₂NR₄R₅ peuvent être obtenus par une méthode analogue à celle décrite dans Pharmazie 1990, 45, 346, ou selon des méthodes décrites dans la littérature ou connues de l'homme du métier.

Les composés de formule générale (I) ou (II) dans laquelle W représente un atome de soufre peuvent, par exemple, être obtenus par réaction des composés de formule générale (I) ou (II) correspondants dans lesquels W représente un atome d'oxygène, avec un réactif tel que le réactif de Lawesson.

Les composés de formule générale (I) dans laquelle R₃ et/ou R₃' représente, indépendamment l'un de l'autre, un atome de fluor peuvent, par exemple, être obtenus par réaction des composés de formule générale (I) correspondants dans laquelle R₃ et/ou R₃' représente, indépendamment l'un de l'autre, un groupe hydroxy, par réaction avec un réactif tel que le trifluorure de diéthylaminosoufre ou selon toutes autres méthodes connues de l'homme de l'art.

Les composés de formule générale (I) dans laquelle R₃ et/ou R'₃ représentent, indépendamment l'un de l'autre, un groupe C₁-C₆-alcoxy, C₃-C₇-cycloalkyloxy ou C₃-C₇-cycloalkyl-C₁-C₃-alkylénoxy, peuvent, par exemple, être obtenus par réaction des composés de formule générale (I) correspondants dans laquelle R₃ et/ou R₃' représente, indépendamment l'un de l'autre, un groupe hydroxy, par réaction avec un réactif tel qu'un halogénure d'alkyle ou un sulfate d'alkyle en présence d'une base telle qu'une solution aqueuse de soude ou du carbonate de potassium ou selon toutes autres méthodes connues de l'homme de l'art.

A chaque étape de la synthèse, on peut protéger divers groupes ou atomes, par exemple un groupe alcool ou amine ou un atome d'azote, selon toutes méthodes connues de l'homme de l'art. On peut ensuite déprotéger les dits-groupes ou atomes pour conduire au composé de formule générale (I), (II) ou (III) selon toutes méthodes connues de l'homme de l'art.

Par exemple, les composés de formule générale (I) pour lesquelles R₃ correspond à un groupe protecteur porté par un atome d'azote, tel qu'un groupe éthoxycarbonyle, terbutyloxycarbonyle ou un groupe benzyloxycarbonyle, peuvent être déprotégés selon des méthodes chimiques connues de l'homme de l'art, pour conduire à des composés de formule générale (I) où R₃ est un atome d'hydrogène.

Les composés de formule générale (I) pour lesquelles R₃ correspond à un groupe hydroxy peuvent être transformés, selon des méthodes chimiques connues de l'homme de l'art, pour conduire à des composés de formule générale (I) où R₃ correspond à un groupe aryle-C(O)-O-, C₁-C₆-alkyle-C(O)-O-, C₃-C₇-Cycloalkyl-C(O)-O-, C₃-C₇-cycloalkyl-C₁-C₃-alkylène-C(O)-O ou C₁-C₆-fluoroalkyle-C(O)-O-.

De même les composés de formule générale (I) pour lesquelles R₃ correspond à un groupe NH₂ peuvent être transformés, selon des méthodes chimiques connues de l'homme de l'art pour conduire à des composés de formule générale (I) où R₃ correspond à un groupe NR₆C(O)R₇ avec R₆ et R₇ tels que définis dans la formule générale de (I).

Les composés de formule générale (I), pour lesquelles R₃ correspond à un groupe hydroxy porté par un carbone asymétrique de configuration déterminé, peuvent être transformés en composés de formule générale (I), pour lesquelles R₃ correspond à un groupe hydroxy, porté par un carbone asymétrique de configuration inversé, par une séquence réactionnelle en deux étapes connue de l'homme de l'art. Dans un premier temps, le groupe hydroxy peut être transformé en groupe ester par réaction avec un acide carboxylique tel que l'acide benzoïque ou l'acide acétique, en présence d'un réactif, tel que l'azodicarboxylate d'éthyle, et d'une phosphine telle que la triphénylphosphine, le tout en solution dans un solvant tel que la tétrahydrofurane. Dans un second temps, le groupe ester ainsi obtenu, peut être hydrolysé, par exemple en présence d'une solution aqueuse de soude, pour conduire aux composés de formule générale (I), pour lesquelles R₃ correspond à un groupe hydroxy porté par un carbone asymétrique de configuration inversé.

Les composés de formule générale (III) sont utiles comme intermédiaires de synthèse des composés da formule (I).

Parmi les composés de formule générale (III), l'invention, selon un autre de ses aspects, a également pour objet les composés de formule générale (IV) 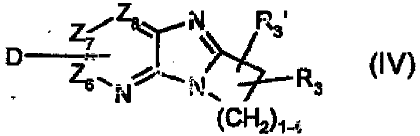 dans laquelle
Z₆, Z₇ et Z₈ représentent, indépendamment l'un de l'autre, un atome de carbone ou un groupe C-R₂ ; l'un des Z₆, Z₇ et Z₈ correspond à un atome de carbone et porte le groupe D ;
D représente un atome d'halogène ou un groupe NH₂ ;
R₂ représente un atome d'hydrogène :
R₃ et R₃', représentent, indépendamment l'un de l'autre, un atome d'hydrogène, un atome d'halogène, un groupe C₁-C₆-alkyle, C₃-C₇cycloalkyle, C₃-C₇-cycloalkyl-C₁-C₃-alkylène, C₁-C₆-fluoroalkyle, hydroxyle, thiol, C₁-C₆-alcoxy, C₃-C₇-cycloalkyloxy, C₃-C₇-cycloalkyl-C₁-C₃-alkylénoxy, aryle-C(O)-O-, C₁-C₆-alkyle-C(O)-O-, C₃-C₇-cycloalkyl-C(O)-O-, C₃-C₇-cycloalkyl-C₁-C₃-alkylène-C(O)-O, C₁-C₆-fluoroalkyle-C(O)-O-, oxo, thio, NR₄R₅, NR₆C(O)R₇, hétéroaryle ou aryle ; les groupes hétéroaryle et aryle étant éventuellement substitués par un ou plusieurs substituants choisis parmi un atome d'halogène, un groupe C₁-C₆-alkyle, C₃-C₇-cycloakyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou bien R₃ et R₃' forment ensemble, avec l'atome de carbone qui les porte, un groupe C₃-C₇-cycloalk-1,1-diyle.
R₄ et R₅, représentent, indépendamment l'un de l'autre, un atome d'hydrogène ou un groupe C₁-C₆-alkyle, C₃-C₇-cycloalkyle, C₃-C₇-cycloalkyl-C₁-C₃-alkylène, aryle-C₁-C₅-alkylène ou aryle, ou R₄ et R₅ forment ensemble, avec l'atome d'azote qui les porte, un groupe azétidine, pyrrolidine, pipéridine, azépine, morpholine, thiomorpholine, pipérazine, homopipérazine, le groupe NR₄R₅ étant éventuellement substitué par un groupe C₁-C₆-alkyle, C₃-C₇-cycloalkyle, C₃-C₇-cycloalkyl-C₁-C₃-alkylène, aryle-C₁-C₅-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂-, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, hydroxy, C₁-C₆-akyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
R₆ et R₇ représentent, indépendamment l'un de l'autre, un atome d'hydrogène, un groupe C₁-C₆-alkyle,C₃-C₇-cycloalkyle, C₃-C₇-cycloalkyl-C₁-C₃-alkylène, aryle-C₁-C₅-alkylène ou aryle ; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ; ou R₆ et R₇ forment ensemble un lactame de 4 à 7 chaînons comprenant l'atome d'azote et le groupe C(O) qui les portent.

Parmi les composés de formule générale (IV), un groupe de composés est constitué par les composés qui répondent à la formule générale (IV') : 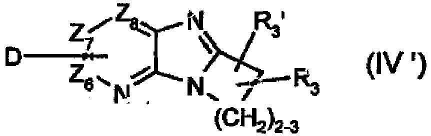 dans laquelle
Z₆, Z₇ et Z₈ représentent, indépendamment l'un de l'autre, un atome de carbone ou un groupe C-R₂ ; l'un des Z₆, Z₇ et Z₈ correspond à un atome de carbone et porte le groupe D ;
D représente un atome d'halogène ou un groupe NH₂ ;
R₂ représente un atome d'hydrogène ;
R₃ et R₃', représentent, indépendamment l'un de l'autre, un atome d'hydrogène, un groupe C₁-C₆-alkyle, hydroxyle, C₁-C₆-alkyle-C(O)-O-, NR₄R₅ ou aryle ;
R₄ et R₅, représentent, indépendamment l'un de l'autre, un atome d'hydrogène ou un groupe C₁-C₆-alkyle.

Les composés de formule (IV) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (IV) peuvent exister à l'état de bases ou de sels d'addition à des acides. De tels sels d'addition font partie de l'invention.

Le schéma 2 illustre la préparation des composés de formule générale (VIII), c'est-à-dire les composés de formule générale (IV), dans laquelle le groupe D est lié au groupe Z₇ qui représente un atome de carbone, et Z₅ et Z₆ représentent un groupe CH. Les composés de formule générale (VIII) peuvent être obtenus, par exemple, par action d'un aminoester de formule générale (V), dans laquelle la chaîne alkyle est éventuellement substituée par R₃ et/ou R₃' tels que définis dans la formule générale (IV) et R représente un groupe C₁-C₆-alkyle, phényle ou benzyle, avec un réactif de formule générale (Vb), dans laquelle R' représente un atome d'halogène, par exemple un atome de brome, ou un groupe nitro, pour conduire à un produit substitué de formule générale (VI).

Lorsque le groupe R' représente un groupe nitro, la réduction des groupes nitro du composé de formule générale (VI) en groupes amino, par exemple en présence d'un catalyseur tel que le palladium sur charbon, sous atmosphère d'hydrogène, en suspension dans un solvant tel que l'éthanol, ou selon toutes méthodes de rédaction connues de l'homme du métier, permet d'accéder au produit de formule générale (VII) dans laquelle D représente un groupe amino.

Lorsque le groupe R' représente un atome d'halogène, par exemple une atome de brome, la réduction du groupe nitro du composé de formule générale (VI) en groupes amine peut être réalisée dans des conditions plus chimiosélectives comme, par exemple, en présence de poudre de fer et d'une solution aqueuse acide telle qu'une solution de chlorure d'ammonium ou selon toutes autres méthodes permettant d'accéder au produit de formule générale (VII) dans lequel D représente un atome d'halogène.

Le composé de formule générale (VII) peut finalement être transformé en composé tricyclique de formule générale (VIII) par cyclisation, par exemple au reflux d'une solution acide telle qu'une solution aqueuse d'acide chlorhydrique.

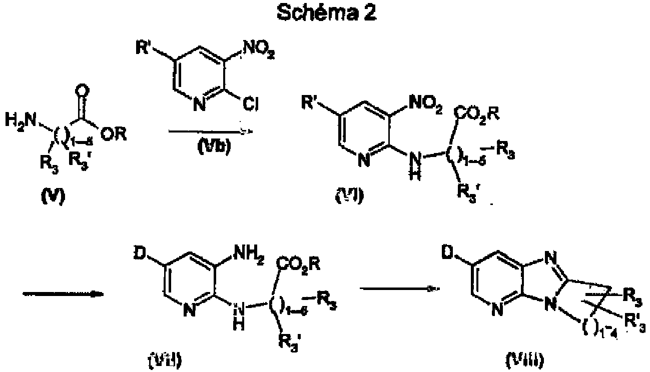

Les composés de formule générale (IV) dans lesquelles le groupe D est lié soit au groupe Z₆ soit au groupe Z₃, lorsque respectivement soit le groupe Z₆ soit le groupe Zₐ représente un atome de carbone, peuvent être obtenus par analogie à la méthode décrite dans le schéma 2.

D'autre part, certains composés de formule générale (IV) peuvent également être obtenus, par exemple, par réaction de la 2-(pyrrolidin-1-yl)-3,5-dinitropyridine (Org. & Biomol. Chem. 2003, 1(6), 1004 - 1011) avec un acide de Lewis tel que le chlorure de zinc, en présence d'un réactif tel que l'anhydride acétique (voir schéma 3). Dans ces conditions, le composé tricyclique de formule générale (IX) est isolé. Le groupe nitro du composé de formule générale (IX) peut ensuite être réduit pour conduire à l'amine de formule générale (IV-b), par exemple en présence d'un catalyseur tel que le palladium sur charbon, sous atmosphère d'hydrogène, on suspension dans un solvant tel que l'éthanol, ou selon toutes méthodes de réduction connues de l'homme du métier.

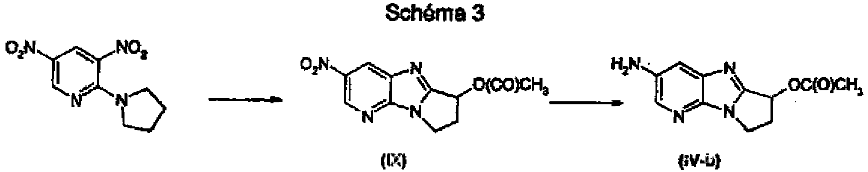

Dans les schémas 2 ot 3, les composés de formule générale (V) et (Vb) et les autres réactifs, quand leur mode de préparation n'est pas décrit, sont disponibles dans le commerce, décrits dans la littérature ou préparés par analogie à des procédés décrits dans la littérature.

Le tableau 1 qui suit illustre les structures chimiques et les propriétés physiques de quelques exemples de composés intermédiaires do formule (IV) selon invention. Dans ce tableau :
- dans la colonne « Sel/base ». « - » représente un composé sous forme de base libre, alors que « HCl » représenté on composé sous forme de chlorhydrate et le rapport entre parenthèses est le rapport (acide : base)

**Tableau 1**

| **N°** | **Structure** | **Sel / base** |
|---|---|---|
| **IV-a** | 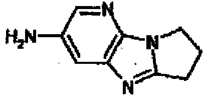 | HCl (2:1) |
| **IV-b** | 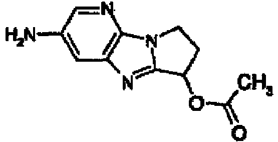 | - |
| **IV-c** | 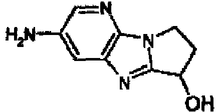 | - |
| **IV-d** | 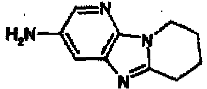 | - |
| **IV-e** | 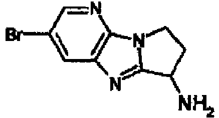 | HCl |
| **IV-f** | 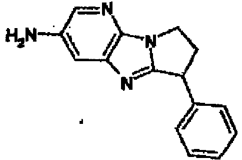 | - |
| **IV-g** | 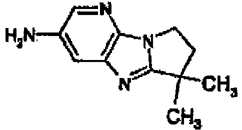 | - |
| **IV-h** | 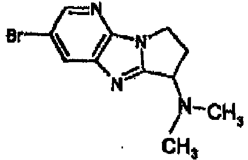 | - |

Les exemples suivants décrivent la préparation de certains composés intermédiaires de formule (IV) conformes à l'invention. Les numéros des composés exemplifiés renvoient à ceux du tableau 1. Les microanalyses élémentaires, les analyses LC-MS (chromatographie liquide couplée à la spectrométrie de masse) les spectres I.R. ou R.M.N. confirment les structures des composés obtenus. Les préparations des composés comportant un carbone chiral ont été mise en oeuvre à partir des réactifs chiraux commerciaux de formule (\/) et le pouvoir rotatoire des composés finaux a été mesuré.

### Exemple A (composé IV-a)

### Chlorhydrate (2:1) de 7,8-dihydro-6H-pyrrolo[2',1':2,3]imidazo[4,5-b]pyridin-3-ylamine

### A.1 4-(3,5-Dinitropyridin-2-yl)aminobutanoate de tert-butyle

A une suspension de 3 g (14,74 mmoles) de 2-chloro-3,5-dinitropyridine dans le dioxane, on ajoute 3,46 g (17,69 mmoles) du chlorhydrate de 4-aminobutanoate de tert-butyle puis 4,21 mL (22,11 mmoles) de triéthylamine. Le mélange réactionnel est agité à 60°C pendant 3 heures.

Après retour à température ambiante, on verse le mélange sur 100 g de glace et on agite vigoureusement jusqu'à fusion complète de la glace. La précipité jaune formé est recueilli par filtration, rincé plusieurs fois à l'eau puis séché à l'étuve sous pression réduite. On obtient 4,65 g de produit sous la forme d'une poudre jaune.

R.M.N. ¹H (DMSO-D₆), δ ppm : 8,3 (s, 1H) ; 9,25 (s, 1H) ; 8,95 (s, 1H) ; 3,7 (q, 2H); 2,3 (t, 2H) ; 1,85 (quint, 2H) ; 1,4 (s, 9H).

### A.2. 4-(3,5-Diaminopyridin-2-yl)aminobutanoate de tert-butyle

Une suspension de 4,2 g (12,87 mmoles) de 4-(3,5-dinitropyridin-2-yl)aminobutanoate de tert-butyle, obtenu dans l'étape précédente, et de 1 g da palladium sur charbon 10% dans 50 mL d'éthanol est agités vigoureusement à température ambiante pendant 3 heures sous 5 atm d'hydrogène.

Après ce temps, le mélange est filtré sur célite en rinçant à l'éthanol. Le filtrat est ensuite concentré sous pression réduite et permet ainsi d'obtenir le 4-(3,5-diamino-pyridin-2-ylamino)butanoate de tert-butyle qui sera utilisé tel quel dans l'étape suivante. R.M.M. ¹H (DMSO D₆), δ (ppm): 6,9 (s, 1H) ; 6,2 (s, 1H) ; 4,7 (m, 1H) ; 4,5 (m, 2H) ; 3,35 (m, 2H) ; 3,2 (m, 2H) ; 2,3 (m, 2H) ; 1,75 (m, 2H) ; 1,4 (s, 9H).

### A.3 Chlorhydrate (2:1) de 7,8-dihydro-6H-pyrrolo[2',1':2,3]imidazo[4,5-b]pyridin-3-ylamine

Une solution de 3 g (11,26 mmoles) de 4-(3,5-diaminopyridin-2-yl)aminobutanoate de tert-butyle, obtenu à l'étape précédente, dans 100 mL d'acide chlorhydrique 6N est portée à reflux pendant 12 heures.

Après ce temps, le mélange est refroidi à température ambiante puis concentré sous pression réduite. Le résidu gommeux résultant est repris dans 50 mL d'éthanol puis concentré sous pression réduite. On répète 3 fois cette opération jusqu'à l'obtention d'une poudre que l'on reprend avec 100 mL d'éther diisopropylique bouillant. La suspension est filtrée à chaud et séchée pour donner 2,56 g de produit sous forme de chlorhydrate.

R.M.N, ¹H (DMSO-D₆), δ ppm : 8,25 (s, 1H) ; 7,7 (s, 1H) ; 6,2 (s large, NH₂) ; 4,3 (t, 2H) ; 3,3 (t, 2H) ; 2,8 (quint, 2H).

### Exemple B (composé IV-b)

### Acétate de 3-amino-7,8-dihydro-6H-pyrrolo[2',1':2,3]imidazo[4,5-b]-pyridin-6-yle

### B.1 Acétate de 3-nitro-7,8-dihydro-6H-pyrrolo[2',1':2,3]imidazo[4,5-b]pyridin-6-yle

On chauffe six heures à reflux un mélange de 1 g (4,2 mmoles) da 2-(pyrrolidin-1-yl)-3,5-dinitropyridine (Org. & Biomol. Chem. 2003, 1(6), 1004 - 1011) et de 0,584; g (4,2 mmoles) de chlorure de zinc dans 10 mL d'anhydride acétique. Après ce temps, on additionne 0,584 g (4,2 mmoles) de chlorure de zinc supplémentaire et on maintient l'agitation à reflux durant dix-huit heures. Après ce temps, le mélange est versé sur 100 g de glace puis extrait trois fois avec 50 mL d'acétate d'éthyle. Les phases organiques sont réunies puis lavées deux fois par 20 mL d'une solution saturée en hydrogénocarbonate de sodium, une fois avec 20 mL d'eau puis une fois avec 50 ml d'une solution saturée en chlorure de sodium, séchées sur sulfate de sodium puis concentrées sous pression réduite. Le produit obtenu est purifié par chromatographie sur colonne de silice en éluant avec un mélange d'acétate d'éthyle et de n-heptane. Le produit résultant est recristallisé dans un mélange d'éther diisopropylique et d'isopropanol. On isole ainsi 0,29 g du composé attendu.

R.M.N. ¹H (DMSO D₆), δ (ppm): 9.28 (s, 1H) ; 8,91 (s, 1H) ; 6,27 (m, 1H) ; 4,42 (m, 1H); 4,3 (m, 1H) ; 3,18 (m, 1H) ; 2,65 (m, 1H) ; 2,11 (s, 3H).

### B.2 Acétate de 3-amino-7,8-dihydro-6H-pyrrolo[2',1':2,3]imidazo[4,5-b]pyridin-6-yle

On agite, 3 heures à 20°C et sous 5 atmosphères d'hydrogène, une suspension de 0,25 g (0,95 mmole) d'acétate de 3-nitro-7,8-dihydro-6*H*-pyrrolo[2',1':2,3]imidazo[4,5-*b*]pyridin-6-yle, obtenue à l'étape précédente et de 0,21 g de Palladium sur charbon à 10% dans 60 mL d'éthanol. Après ce temps, le mélange est filtré sur un tampon de célite puis concentré sous pression réduite. On obtient 0,22 g du produit attendu qui sera utilisé tel quel dans la suite de la synthèse.

R.M.N. ¹H (DMSO D₆), δ (ppm): 7,85 (s, 1H) ; 7,2 (s, 1H) ; 6,1 (m, 1H) ; 5.05 (s, 2H) ; 4,2 (m, 1H) ; 4,1 (m, 1H) ; 3,1 (m, 1H) ; 2,55 (m, 1H) ; 2.11 (s, 3H).

### Exemple C (composé IV-c)

### (-)-3-Amino-7,8-dihydro-6H-pyrrolo[2',1':2,3]imidazo[4,5-b]pyridin-6-ol

### C.1 4-[(3,5-Dinitropyridin-2-yl)amino]-2-hydroxybutyrate de méthyle

On chauffe quatre heures à 65°C un mélange de 2,8 g (13,48 mmoles) de 2-chloro-3,5-dinitropyridine, 2,75 g (16,2 mmoles) de chlorhydrate de (S)-4-amino-2-hydroxybutyrate de méthyle et 3,41 g (2,5 mmoles) de triéthylamine dans 50 mL de dioxane.

Après ce temps, on concentre sous pression réduite le milieu réactionnel que l'on reprend avec 50 mL d'eau. On extrait le mélange avec deux fois 50 mL de dichlorométhane. Les phases organiques sont rassemblées, lavées avec 50 ml d'une solution saturée en carbonate de sodium, séchées sur sulfate de sodium puis concentrées sous pression réduite. L'huile obtenue est purrfiée par chromatographie sur colonne de silice en éluant avec un mélange de dichlorométhane et de méthanol. On isole ainsi 2,1 g du produit attendu que l'on utilise tel quel dans la suite de la synthèse.
MS: [MH]⁺ = 301
R.M.N. 1H CCDCl₃), δ (ppm): 9,33 (d, 1H) ; 9,3 (d, 1H) ; 9,21 (singulet élargi, 1H) ; 4,42 (m, 1H) ; 4,01 (m, 2H) ; 3,88 (s, 3H) ; 3,21 (d, 1H) ; 2,37 (m, 1H) ; 2,12 (m, 1H).

### C.2 4-[(3,5-Diaminopyridin-2-yl)amino]-2-hydroxybutyrate de méthyle

On agite vigoureusement, pendant 7 heures à 20°C sous 5 atmosphère d'hydrogène une suspension de 1,9 g (6,33 mmoles) de 4-[(3,5-Dinitropyridin-2-yl)amino]-2-hydroxybutyrate de méthyle, obtenu à l'étape précédente et 0,1 g de palladium sur charbon à 10% dans 100 mL de méthanol. Après ce temps, le mélange est filtré sur un tampon de célite puis concentré sous pression réduite. On récupère ainsi 1,45 g du produit attendu que l'on utilise tel quel dans la suite de la synthèse.

### C.3 (-)-3-Amino-7,8-dihydro-6H-pyrrolo[2',1':2,3]imidazo[4,5-b]pyridin-6-ol

On agite quinze heures à reflux une solution de 1,4 g (5,83 mmoles) de 4-[(3,5-diaminopyridin-2-yl)amino]-2-hydroxybutyrate de méthyle, obtenu à l'étape précédente, dans 50 mL d'acide chlorhydrique 6N. Après ce temps, le mélange est concentré sous pression réduite jusqu'à un volume de 10 mL. Le pH de la solution est ajusté à pH 10 par ajouts successifs de carbonate de sodium puis le mélange est concentré sous pression réduite et le solide résultant est trituré avec 100 mL d'une solution à 10% de méthanol dans le dichloromémane. Le mélange est filtré et l'opération est répétée trois fois. Les filtrats sont réunis, concentrés sous pression réduite et le produit résultant est purifié par chromatographie sur colonne de silice en éluant avec un mélange de dichlorométhane et de méthanol. On isole ainsi 0,3 g du produit attendu.
PF = 229 - 230 °C
[α]_{D}²⁰ = -17,43°, c = 0,175 g/100 mL ; DMSO
MS: [MH]⁺ = 191
R.M.N. 1 H (DMSO D₆), δ (ppm): 7,77 (s, 1H) ; 7,19 (s, 1H) ; 5,80 (d, 1H) ; 5,08 (m, 1 H) ; 4,93 (pic élargi, 2H) ; 4,17 (m, 1H) ; 3,99 (m, 1H) ; 2,91 (m, 1 H) ; 2,39 (m, 1H).

### Exemple D (composé IV-d)

### 6,7,8,9-Tétrahydroimidazo[1,2-a:5,4-b']dipyridin-3-amine

### D.1 5-[(3,5-Dinitropyridin-2-yl)amino]pentanoate d'éthyle

On procède de façon analogue à la méthode décrite à l'étape C.1, à partir de 1,2 g (5,9 mmoles) de 2-chloro-3,5-dinitropyridine, de 1,03 g (7,07 mmoles) de 5-aminovalérate d'éthyle et de 0,9 g (8,84 mmoles) de triéthylamine dans 20 mL de dioxane. On isole ainsi 1,4 g de produit attendu.
PF = 70-72°C
MS: [MH]⁺ = 313
R.M.N. 1H (CDCl₃), δ (ppm): 9,18 (m, 2H) ; 8,73 (pic élargi, 1H) ; 4,08 (q, 2H) ; 3,70 (m, 2H) ; 2,32 (m, 2H) ; 1,70 (m, 4H) ; 1,19 (t, 3H).

### D.2 5-[(3,5-Diaminopyridin-2-yl)amino]pentanoate d'éthyle

On procède de façon analogue à la méthode décrite à l'étape C.2, à partir de 1,35 g (4,32 mmoles) du composé préparé à l'étape précédente et 100 mg de palladium sur charbon à 10% en suspension dans 100 mL de méthanol. On isole ainsi 1,1 g du produit attendu, utilisé tel quel dans la suite de la synthèse.

### D.3 6,7,8,9-Tétrahydroimidazo[1,2-a:5,4-b]dipyridin-3-amine

On procède de façon analogue à la méthode décrite à l'étape C.3, à partir de 1,1 g (4,36 mmoles) du produit obtenu à l'étape précédente, en solution dans 30 mL d'acide chlorhydrique 6N, Le produit obtenu est purifié par chromatographie sur colonne d'alumine neutre en éluant avec un mélange de dichlorométhane et de méthanol. On isole ainsi 60 mg du produit attendu utilisé tel quel dans la suite de la synthèse.
MS: [MH]⁺ = 189
R.M.N. 1 H (CDCl₃), δ (ppm): 7,79 (s, 1H) ; 7,20 (s, 1H) ; 4,09 (t, 2H) ; 3,49 (pic élargi, 2H) ; 2,88 (t, 2H) ; 1,99 (m, 4H)

### Exemple E (composé IV-e)

### Chlorhydrate de 3-Bromo-7,8-dihydro-6H-pyrrolo[2',1':2,3]imidazo[4,5-b]pyridin-6-amine

### E.1 4-[(5-Bromo-3-nitropyridin-2-yl)amino]-2-tert-butoxycarbonylamino-butanoate de tert-butyle.

On procède de façon analogue à la méthode décrite à l'étape C.1, à partir de 2 g (8,42 mmoles) de 5-bromo-2-chloro-3-nitropyridine, 3,14 g (10,11 mmoles) de chlorhydrate de (S)-4-amino-2-tert-butoxycarbonylamino-butanoate de tert-butyle et 2,31 g (16,85 mmoles) de triéthylamine dans 30 mL de dioxane. On chauffe cette fois le mélange durant 12 heures. On isole ainsi 3,71 g du produit attendu utilisé tel quel dans la suite de la synthèse.
MS: [MH]⁺ = 475
R.M.N. 1H (DMSO D₆), δ (ppm): 8,59 (s, 2H) ; 8,52 (m, 1H) ; 7,18 (d, 1H) ; 3,91 (m, 1H) ; 3,68 (m, 2H) ; 1,94 (m, 2H) 1,4 (s, 18H).

### E.2 4-[(3-amino-5-bromo-pyridin-2-yl)amino]-2-tert-butoxycarbonylamino-butanoate de tert-butyle

On chauffe à reflux deux heures une suspension de 3,5 g de 4-[(5-Bromo-3-nitro-pyridin-2yl)amino]-2-tert-butoxycarbonylamino-butanoate de tert-butyle, 1,23 g (22 mmoles) de poudre de fer et 20 g (0,373 mole) de chlorure d'ammonium dans 40 mL d'un mélange 1:1 d'éthanol et de tétrahydrofurane. Après ce temps, le mélange réactionnel est filtré sur un tampon de célite que l'on rince avec 100 mL d'éthanol. On concentre le filtrat sous pression réduite puis on reprend avec 20 mL d'eau. Le mélange est alors extrait avec trois fois 50 mL d'acétate d'éthyle. Les phases organiques sont rassemblées, lavées avec 50 mL d'une solution aqueuse saturée en chlorure de sodium, séchées sur sulfate de sodium puis concentrées sous pression réduite. On récupère une huile qui est purifiée par chromatographie sur colonne de silice en éluant avec un mélange de dichlorométhane et de méthanol. On isole le ainsi 2,93 g du produit attendu sous la forme d'un solide, utilisé tel quel dans la suite de la synthèse.
MS: [MH]⁺ = 445
R.M.N. 1 H (DMSO D₆), δ (ppm): 7,39 (s, 1H) ; 7,21 (d, 1H) ; 6,81 (s, 1H) ; 5,70 (pic élargi, 1H) ; 5,07 (pic élargi, 2H) ; 3,91 (m, 1H) ; 3,32 (m, 2H) ; 1,98 (m, 1H) ; 1,81 (m, 1H) ; 1,4 (s, 18H).

### E.3 Chlorhydrate de (+)-3-Bromo-7,8-dihydro-6H-pyrrolo[2',1':2,3]imidazo[4,5-b]pyridin-6-amine (IV-e)

On procède de façon analogue à la méthode décrite à l'étape C.3, à partir de 2,75 g (5,56 mmoles) de 4-(3-amino-5-bromo-pyridin-2-ylamino)-2-tert-butoxycarbonylamino-butanoate de tert-butyle et de 40 mL d'acide chlorhydrique 6N. Après refroidissement à 20°C, le mélange réactionnel est successivement concentré sous pression réduite, repris avec 50 mL d'éthanol et concentré sous pression réduite pour obtenir un solide que l'on triture avec de l'éther isopropylique chaud. On récupère, par filtration, 1,31 g d'une poudre que l'on utilise telle quelle dans la suite de la synthèse.
MS: [MH]⁺ = 253
[α]_{D}²⁰ = +23,81°, c = 0,113 g/100 mL ; MeOH
R.M.N. ¹H (DMSO D₆), δ (ppm): 9,11 (plc élargi, 2H) ; 8,49 (s, 1H) ; 8,43 (s, 1H) ; 4,96 (m, 1H) ; 4,4 (m, 1H) ; 4,23 (m, 1H) ; 3,12 (m, 1H) ; 2,73 (m, 1H).

(+)-3-Bromo-7,8-dihydro-6*H*-pyrrolo[2',1':2,3]imidazo[4,5-*b*]pyridin-6-amine (IV-e) L'amine (IV-e) sous forme de base a été obtenue de façon classique, à partir d'une solution du chlorhydrate dans l'eau, en ajustant le pH par ajouts successifs d'une solution aqueuse d'ammoniaque, puis par extraction avec une solution d'acétate d'éthyle et concentration sous pression réduite.
R.M.N. ¹H (DMSO D₆), δ (ppm): 8,4 (d, 1H) ; 8,25 (d, 1H) ; 4,4 (m, 1H) ; 4,25 (m, 1H) ;3 4,1 (m, 1H) ; 2,9 (m, 1H) ; 2,3 (m, 1H) ; 2,2 (pic élargi, 2H).
[α]_{D}²⁰ = +0,568°, c = 1,004 g/100 mL ; MeOH

### Exemple F (composé IV-f)

### 6-Phényl-7,8-dihydro-6H-pyrrolo[2',1':2-3]imidazo[4,5-b]pyridin-3-amine

### F.1 Chlorhydrate de 4-amino-2-phényl-butyrate de méthyle

Un mélange de 1 g (4,64 mmoles) de chlorhydrate d'acide 4-amino-2-phényl-butyrique et de 0,67 mL (5,1 mmoles) de chlorure de triméthylsilyle dans 50 mL de méthanol est chauffé 2 heures à reflux. Le mélange est ensuite agité 12 heures à 20°C puis concentré sous pression réduite, trituré dans 50 mL d'éther éthylique puis filtré. On recueille ainsi 0,9 g du produit attendu sous la forme d'un solide blanc.
MS: [MH]⁺ =194

### F.2 4-(3,5-Dinitropyridin-2-ylamino)-2-phényl-butyrate de méthyle

On procède de façon analogue à la méthode décrite à l'étape C.1, à partir de 0,8 g (3,93 mmoles) de 2-chloro-3,5-dinitropyridine et de 1 g (4,32 mmoles) de 4-amino-2-phenyl-butyrate de méthyle. On isole 1,1 g de produit attendu.

R.M.N. ¹H (CDCl₃), δ (ppm): 9,29 (m, 2H) ; 8,79 (pic élargi, 1H) ; 7,38 (m, 5H) ; 3,8 (m, 3H) ; 3,74 (s, 3H) ; 2,6 (sext., 1H) ; 2,31 (sext., 1H).

### F.3 4-(3,5-Diaminopyridin-2-ylamino)-2-phényl-butyrate de méthyle

On procède de façon analogue à la méthode décrite à l'étape C.2, à partir de 1,1 g (3,05 mmoles) de 4-(3,5-dinitropyridin-2-ylamino)-2-phényl-butyrate de méthyle. On prépare ainsi 0.9 g du produit attendu que l'on utilise tel quel dans la suite de la synthèse.
MS: [MH]⁺ = 301

### F.4 6-Phényl-7,8-dihydro-6H-pyrrolo[2',1':2,3]imidazo[4,5-b]pyridin-3-amino (IV-f)

On procède de façon analogue à la méthode décrite à l'étape C.3, à partir de 0.9 g (3,3 mmoles) de 4-(3,5-Diaminopyridin-2-ylamino)-2-phényl-butyrate de méthyle. On isole ainsi 0,88 g du produit attendu que l'on utilise tel quel dans la suite de la synthèse.
MS: [MH]⁺ = 251
R.M.N. ¹H (CDCl₃), δ (ppm): 7,78 (d, 1H) ; 7,2 (m, 6H) ; 4,43 (m, 1H) ; 4,29 (m, 1H) ; 4,11 (m, 1H) ; 3,5 (pic élargi, 2H) ; 3,09 (m, 1H) ; 2,62 (m, 1H).

### Exemple G (composé IV-g)

### 6,6-Dimethyl-7,8-dihydro-6H-pyrrolo[2',1':2,3]imido[4,5-b]pyridin-3-amine (IV-g)

### G.1 Chlorhydrate de 4-amino-2,2'-diméthylbutyrate de méthyle

On procède de façon analogue à la méthode décrite à l'étape F.1, à partir de 1 g (5,97 mmoles) d'acide 4-amino-2,2'-diméthylbutyrique. On obtient ainsi 1,04 g de produit attendu.

R.M.N. ¹H (DMSO D₆), δ (ppm): 8,03 (pic élargi, 2H) ; 3,67 (s, 3H) ; 2,71 (m, 2H) ; 1,8 (m, 2H) ; 1,15 (s, 6H).

### G.2 4-[(3,5-Dinitropyridin-2-yl)amino]-2,2'-diméthylbutyrate de méthyle

On procède de façon analogue à la méthode décrite à l'étape C-1, à partir de 0.9 g (4,42 mmoles) de 2-chloro-3,5-dinitropyridine et 0,96 g (5,31 mmoles) de chlorhydrate de 4-amino-2,2-diméthylbutyrate de méthyle, préparé à l'étape précédente. Après 5 heures d'agitation à 70°C, le mélange réactionnel refroidi est versé sur 100 mL d'eau glacée. La solution résultante est extraite trois fois avec 50 mL de dichlorométhane. Les phases organiques sont réunies, lavées avec 50 mL d'eau, séchées sur sulfate de sodium puis concentrées sous pression réduite. On obtient ainsi 1,5 g du composé attendu que l'on utilise tel quel dans la suite de la synthèse.
R.M.N. ¹H (CDCl₃), δ (ppm): 9,33 (d, 1H) ; 9,29 (d, 1H) ; 8,82 (pic élargi, 1H) ; 3,87 (m, 2H) ; 3,78 (s, 3H) ; 2,08 (m, 2H) ; 1,35 (s, 6H).

### G.3 4-[(3,5-Diaminopyridin-2-yl)amino]-2,2'-diméthylbutyrate de méthyle

On procède de façon analogue à la méthode décrite à l'étape C2, à partir da 1,05 g (3,36 mmoles) de 4-[(3,5-Dinitropyridin-2-yl)amino]-2,2'-diméthylbutyrate de méthyle obtenu à l'étape précédente. On isole ainsi 1 g du produit attendu.

R.M.M. ¹H (DMSO D₆), δ (ppm): 6,79 (d, 1H) ; 6,31 (d, 1H) ; 5,5-4,2 (pic élargi, 5H) ; 3,61 (s, 3H) ; 3,15 (m, 2H) ; 1,82 (m, 2H) ; 1,19 (s, 6H).

### G.4 6,6-Diméthyl-7,8-dihydro-6H-pyrrolo[2',1':2,3]imidazo[4,5-b]pyridin-3-amino (IV-g)

On procède de façon analogue à la méthode décrite à l'étape C.3, à partir de 1 g (3,96 mmoles) de 4-[(3,5-Diaminopyridin-2-yl)amino-]2,2'-diméthylbutyrate de méthyle obtenu à l'étape précédente. On isole 0,400 g de produit attendu.
R.M.N. ¹H (CDCl₃), δ (ppm): 8,04 (d, 1H) ; 7,47 (d, 1H) ; 4,11 (m, 1H) ; 2,48 (m, 2H) ; 1,41 (s, 6H).
MS: [MH]⁺ = 203

### Exemple H (composé IV-h)

### N,N-Diméthyl-3-Bromo-7,8-dihydro-3H-pyrrolo[2',1':2,3]imidazo[4,5-b]pyridin-6-amine (IV-h)

Une solution de 0,5 g (1,5 mmole) de composé IV-e sous forme de chlorhydrate dans 10 mL de méthanol est éluée sur une cartouche échangeuse d'ions. On isole, après concentration sous pression réduite de la solution éluée, 0,37 g de 3-Bromo-7,8-dihydro-6*H*-pyrrolo[2',1';2,3]imidazo[4,5-*b*]pyridin-6-amine que l'on reprend dans 10 mL d'acétonitrile. On ajoute ensuite 0,52 mL (6,91 mmoles) d'une solution aqueuse de formaldéhyde à 37%. Le mélange réactionnel est agité 20 minutes puis on ajoute 0,183 g (2,77 mmoles) de cyanoborohydrure de sodium. Après 20 minutes d'agitation supplémentaire, on additionne 0,5 mL d'acide acétique glacial et on agite à 20°C pendant trois heures. La solution est ensuite diluée avec 30 mL d'un mélange de dichlorométhane et de méthanol. La phase organique est séparée, lavée trois fois avec 20 mL de soude 1M, séchée sur sulfate de sodium puis concentrée sous pression réduite. On obtient ainsi 0,292 g du produit attendu que l'on utilise tel quel dans la suite de la synthèse.
MS: [MH]⁺ = 281
[α]_{D}²⁰ = non déterminé

Les exemples suivants décrivent la préparation de certains composés conformes à l'invention. Ces exemples ne sont pas limitatifs et ne font qu'illustrer la présente invention. Les numéros des composés exemplifiés renvoient à ceux du tableau 2. Les microanalyses élémentaires, les analyses LC-MS (chromatographie liquide couplée à la spectrométrie de masse) et / ou les spectres I.R. ou R.M.N. confirment les structures des composés obtenus. Le pouvoir rotatoire ou la pureté énantiomérique associée à un temps de rétention des composés finaux comportant un carbone chiral ont été mesurés.

### Exemple 1 (Composé N°1)

### N-(7,8-Dihydro-6H-pyrrolo[2',1':2,3]imidazo[4,5-b]pyridin-3-yl)-5-fluoro-1-(3-fluorobenzyl)-1H-indole-2-carboxamide

### 1.1 Acide 5-fluoro-1-(3-fluorobenzyl)-1H-indole-2-carboxylique

On ajoute une solution aqueuse de soude, préparée à partir de 1,15 g (28,92 mmoles) de pastille de soude dans 50 mL d'eau, à une solution de 7,6 g (24,10 mmoles) de 5-fluoro-1-(3-fluorobenzyl)-1*H*-indole-2-carboxylate d'éthyle (WO2006/024776) dans 241 mL d'éthanol. Le mélange est chauffé durant deux heures puis concentré sous pression réduite. Le solide résultant est repris dans 200 mL d'eau. La solution est lavée avec deux fois 100 mL d'éther éthylique, acidifiée par ajouts successifs de petites quantités d'acide chlorhydrique concentré puis extraite avec 200 mL d'acétate d'éthyle. La phase organique est finalement lavée deux fois avec 100 mL d'eau, une fois avec 50 ml d'une solution saturée en chlorure de sodium, séchée sur sulfate de magnésium et concentrée sous pression réduite. On obtient, après séchage à 50°C sous pression réduite, 6,4 g du produit attendu sous la forme d'un solide qui sera utilisé tel quel dans la suite de la synthèse.

### 1.2 N-(7,8-Dihydro-6H-pyrrolo[2',1':2,3]imidazo[4,5-b]pyridin-3-yl)-5-fluoro-1-(3-fluorobenzyl)-1H-indole-2-carboxamide (Composé n°1)

On agite durant 15 minutes à 20°C une solution de 0,5 g (1,74 mmole) d'acide 5-fluoro-1-(3-fluorobenzyl)-1*H*-indole-2-carboxylique, préparé à l'étape 1.1, de 0,33 g (1,74 mmole) de *N*-(3-diméthylaminopropyl)-*N*'-éthylcarbodiimide et de 0,23 g (1,74 mmole) de *N*-1-hydroxybenzotriazole dans 10 mL de diméthylformamide. On ajoute ensuite eu milieu réactionnel 0,516 g (2,09 mmoles) du composé IV-a ainsi que 0,29 mL (2,09 mmoles) de triéthylamine. Le mélange réactionnel est ensuite agité 12 heures à 20°C puis concentré sous pression réduite, versé sur 50 mL d'eau et extrait trois fois avec 50 mL d'acétate d'éthyle. Les phases organiques sont réunies puis lavées deux fois par 20 mL d'une solution saturée en hydrogénocarbonate de sodium, une fois avec 20 ml. d'eau puis une fois avec 50 ml d'une solution saturés en chlorure de sodium, séchées sur sulfate de sodium puis concentrées sous pression réduite. Le produit obtenu est purifié par chromatographie sur colonne de silice en éluant avec un mélange de dichlorométhane et da méthanol. On isole ainsi 0,31 g du composé attendu.
Point de fusion: 227 - 229 °C
MS: [MH]⁺ = 444
R.M.N. ¹H (DMSO D₆), δ (ppm): 10,6 (s, 1H) ; 8,63 (s, 1H), 8,3 (s, 1H) ; 7,59 (m, 2H) ; 7,48 (s, 1H) ; 7,33 (m, 1H) ; 7,18 (m, 1H) ; 7,05 (m, 1H) ; 6,92 (m, 2H) ; 5,91 (s, 2H) ; 4,15 (t, 2H) ; 3 (t, 2H) ; 2,68 (m, 2H).

### Exemple 2 (Composé N°3)

### N-(6-Acétoxy-7,8-dihydro-6H-pyrrolo[2',1':2,3]imidazo[4,5-b]pyridin-3-yl)-5-fluoro-1-(3-fluorobenzyl)-1H-indole-2-carboxamide

On agite durant 15 minutes à 20°C une solution de 0,25 g (0,87 mmole) d'acide 5-fluoro-1-(3-fluorobenzyl)-1*H*-indole-2-carboxylique, préparé à l'étape 1.1, de 0,166 g (0,87 mmole) de *N*-(3-diméthylaminopropyl)-*N'*-éthylcarbodiimide et de 0,117 g (0,87 mmole) de *N*-1-hydroxybenzotriazole dans 3 mL de diméthylformamide. On ajoute ensuite au milieu réactionnel 0,245 g (1,04 mmole) de 6-acétoxy-7,8-dihydro-6*H-*pyrrolo[2',1':2,3]imidazo[4,5-*b*]pyridin-3-amine (composé IV-b), solubilisé dans 3 mL de diméthylformamide. Le mélange réactionnel est ensuite agité 16 heures à 20°C puis concentré sous pression réduite, versé sur 50 mL d'eau et extrait trois fois avec 50 mL d'acétate d'éthyle. Les phases organiques sont réunies puis lavées deux fois par 20 mL d'une solution saturée en hydrogénocarbonate de sodium, une fois avec 20 mL d'eau puis une fois avec 50 ml d'une solution saturée en chlorure de sodium, séchées sur sulfate de sodium puis concentrées sous pression réduite. Le produit obtenu est purifié par chromatographie sur colonne de silice en éluant avec un mélange de dichlorométhane et de méthanol. Le produit ainsi purifié est finalement trituré dans l'éther diisopropylique chaud et on recueille par filtration à chaud 0,27 g du produit attendu.
Point de fusion: 139 - 141 °C
MS: [MH]⁺ = 502
R.M.N. ¹H (DMSO D₆), δ (ppm): 10,69 (s, 1H) ; 8,69 (s, 1H) ; 8,44 (s, 1H) ; 7,61 (m, 2H); 7,5 (s, 1H) ; 7,35 (m, 1H) ; 7,19 (m, 1H) ; 7,05 (m, 1H) ; 6,92 (m, 2H) ; 6,18 (m, 1H) ; 5,91 (s, 2H) ; 4,33 (m, 1H) ; 4,22 (t, 1H) ; 3,17 (m, 1H) ; 2,6 (m, 1H) ; 2,1 (s, 3H).

### Exemple 3 (Composé N°3)

### N-(6-Hydroxy-7,8-dihydro-6H-pyrrolo[2',1':2,3]imidazo[4,5-b]pyridin-3-yl)-5-fluoro-1-(3-fluorobenzyl)-1H-indole-2-carboxamide

On agite deux heures à 20°C une suspension de 0,2 g (0,4 mmole) de composé n°2, préparé dans l'exemple n°2, et de 0,225 g (1,6 mmole) de carbonate de césium dans 5 mL de méthanol. Après ce temps, le mélange réactionnel est concentré sous pression réduite, versé sur 50 mL d'eau et extrait trois fois avec 20 mL de dichlorométhane. Les phases organiques sont réunies puis lavées une fois avec 20 mL d'eau puis une fois avec 50 ml d'une solution saturée en chlorure de sodium, séchées sur sulfate de sodium puis concentrées sous pression réduite. Le produit obtenu est purifié par chromatographie sur colonne de silice en éluant avec un mélange de dichlorométhane et de méthanol puis recristallisé avec un mélange d'éthanol et d'eau. On isole ainsi 110 mg du produit attendu.
Point de fusion: 288 - 290 °C
MS: [MH]⁺ = 460
R.M.N. 1H (DMSO D6), δ (ppm): 8,62 (s, 1H) ; 8,42 (s, 1H) ; 7,61 (m, 2H) ; 7,48 (s, 1H) ; 7,34 (m, 1H) ; 7,19 (m, 1H) ; 7,06 (m, 1H) ; 6,92 (m, 2H) ; 5,99 (d, 1H) ; 5,91 (s, 2H) ; 5,17 (m, 1H) ; 4,29 (m, 1H) ; 4,1 (m, 1H) ; 2,95 (m, 1H) ; 2,45 (m, 1H).

### Exemple 4 (Composé N°23)

### N-(6-Hydroxy-7,8-dihydro-6H-pyrrolo[2',1':2,3]imidazo[4,5-b]pyridin-3-yl)-5-trifluorométhyl-1-(3-trifluorobenzyl)-1H-indole-2-carboxamide

On procède de façon analogue à la méthode décrite à l'étape 1.2, à partir de 0,4 g (1,18 mmole) d'acide 5-trifluorométhyl-1-(3-fluorobenzyl)-1*H*-indole-2-carboxylique (WO2006072736), de 0,29 g (1,54 mmole) de composé (IV-c), de 0,25 g (1,3 mmole) de chlorhydrate de *N*-(3-diméthylaminopropyl)-*N'*-éthylcarbodiimide et de 0,18 g (1,3 mmole) de *N*-1-hydroxybenzotriazole dans 12 mL de diméthylformamide. On obtient ainsi 460 mg du produit attendu.
PF = 267 - 268 °C
MS: [MH]⁺ = 510
Pureté énantiomérique = 94% ; Temps de rétention = 14,5 mn
R.M.N. ¹H (DMSO D₆), δ (ppm): 10,79 (s, 1H) ; 8,61 (s, 1H) ; 8,41 (s, 1H) ; 8,24 (s, 1 H) ; 7,31 (d, 1H) ; 7,65 (s, 1H) ; 7,61 (m, 1H) ; 7,36 (m, 1H) ; 7,09 (m, 1H) ; 6,96 (d, 2H) ; 6 (s, 2H) ; 5,18 (m, 1H) ; 4,29 (m, 1H) ; 4,12 (m, 1H) ; 2,97 (m, 1H) ; 2,43 (m, 1 H).

### Exemple 5 (Composé N°37)

### N-(6,7,8,9-tétrahydro-imidazo[1,2-a:5,4-b']dipyridin-3-yl)-5-trifluorométhyl-1-(3-fluorobenzyl)-1H-indole-2-carboxamide

On procède de façon analogue à la méthode décrite à l'étape 1.2, à partir de 0,06 g (0,18 mmole) d'acide 5-trifluorométhyl-1-(3-fluorobenzyl)-1*H*-indole-2-carboxylique (WO2006072736), de 0,05 g (0,27 mmole) de composé (IV-d), de 0,038 g (0,2 mmole) de chlorhydrate de *N*-(3-diméthylaminopropyl)-*N'*-éthylcarbodiimide et de 0,027 g (0,2 mmole) de *N-*1-hydroxybenzotriazole dans 2 mL de diméthylformamide. On obtient ainsi 70 mg du produit attendu.
PF = 262 - 263 °C
MS: [MH]⁺ = 508
R.M.N. 1H (DMSO D₆), δ (ppm): 10,75 (s, 1H) ; 8,55 (d, 1H) ; 8,30 (d, 1H) ; 8,22 (s, 1H) ; 7,81 (d, 1H) ; 7,62 (s, 1H) ; 7.59 (dxd, 1H) ; 7,34 (q, 1H) ; 7,07 (txd, 1H) ; 6,94 (d, 2H) ; 5,99 (s, 2H) ; 4,15 (m, 2H) ; 3,01 (m, 2H) ; 2,09 (m, 2H) ; 1,65 (m, 2H).

### Exemple 6 (Composé N°30)

### N-(6-Phényl-7,8-dihydro-6H-pyrrolo[2',1':2,3]imidazo[4,5-b]pyridin-3-yl)-5-trifluorométhyl-1-(3-fluorobenzyl)-1H-indole-2-carboxamide

On agite 30 minutes à 20°C un mélange de 50 mg (0,15 mmole) d'acide 5-trifluorométhyl-1-(3-fluorobenzyl)-1*H*-indole-2-carboxylique (WO2006072736), de 44,5 mg (0,18 mmole) de composé (IV-f) et de 29 mg (0,18 mmole) de diéthylcyanophosphonate en solution dans 6 mL de diméthylformamide. On ajoute ensuite 22,5 mg (0,22 mmole) de triéthylamine puis on agite le mélange 12 heures à 20°C. Après ce temps, le mélange est concentré sous pression réduite, repris dans 50 mL d'eau puis extrait trois fois avec 50 mL d'acétate d'éthyle. Les phases organiques sont réunies, lavées avec 20 mL d'eau puis séchées sur sulfate de sodium et concentrées sous pression réduite.

Le produit résultant est ensuite purifié par chromatographie sur colonne de silice en éluant avec un mélange de dichlorométhane et de méthanol. On isole ainsi 75 mg du produit attendu.
PF = 129 - 130 °C
MS: [MH]⁺ = 570
R.M.N. ¹H (DMSO D₆), δ (ppm): 10,75 (s, 1H) ; 8,62 (s, 1H) ; 8,31 (s, 1H) ; 8,21 (s, 1H) ; 7,81 (d, 1H) ; 7,65 (s, 1H) ; 7,61 (d, 1H) ; 7,4 (m, 5H) ; 7,32 (m, 1H) ; 7,08 (m, 1H) ; 6,93 (d, 2H) ; 5,98 (s, 2H) ; 4,68 (m, 1H) ; 4,41 (m, 1H) ; 4,22 (m, 1H) ; 3,2 (m, 1H) ; 2,68 (m, 1H).

### Exemple 7 (Composé N°31)

### N-(6,6-Diméthyl-7,8-dihydro-6H-pyrrolo[2',1':2,3]imidazo[4,5-b]pyridin-3-yl)-5-trifluorométhyl-1-(3-fluorobenzyl)-1H-indole-2-carboxamide

On procède de façon similaire à l'exemple 6 à partir de 0,2 g (0,59 mmole) d'acide 5-trifluorométhyl-1-(3-fluorobenzyl)-1*H*-indole-2-carboxylique (WO2006072736) et de 0,17 g (0,71 mmole) de composé (IV-h). On isole ainsi 25 mg du produit attendu.
PF = 212 - 213 °C
MS: [MH]⁺ = 522
R.M.M. 1H (DMSO D₆), δ (ppm): 10,7 (s, 1H) ; 8,58 (d, 1H) ; 8.29 (d, 1H) ; 8,22 (s, 1H) ; 7,83 (d, 1H) ; 7,63 (s, 1H) ; 7,59 (m, 1H) ; 7,35 (m, 1H) ; 7,07 (m, 1H) ; 6,96 (m, 2H) ; 5,96 (s, 2H) ; 4,2 (m, 2H) ; 3,2 (m, 1H) ; 2,49 (m, 1 H) ; 1,39 (s, 6H).

### Exemple 8 (Composé N°32)

### N-(6-Hydroxy-7,8-dihydro-6H-pyrrolo[2',1':2,3]imidazo[4,5-b]pyridin-3-yl)-5-trifluorométyl-1-(3-fluorobenzyl)-1H-indole-2-carboxamide

### 8.1 Pyridine-2-carboxylate de 3-[[[1-(3-fluorobenzyl)-5-trifluorométhyl-1H-indol-2-yl]carbonyl]amino]-7,8-dihydro-6H-pyrrolo[2',1':2,3]imidazo[4,5-b]pyridin-6-yle

A un mélange, agité à -20°C sous atmosphère inerte, de 0,12 g (0,24 mole) de (-)-*N*-(6-hydroxy-7,8-dihydro-6*H*-pyrrolo[2',1':2,3]imidazo[4,5-*b*]pyridin-3-yl)-5-trifluorométhyl-1-(3-fluorobenzyl)-1*H*-indole-2-carboxamide (composé n°26), de 0,116 g (0,94 mole) d'acide picolinique et de 0,247 g (0,94 mole) de triphénylphosphine dans 5 mL de tétrahydrofurane, sont ajoutés goutte à goutte 0,19 g (0,94 mmole) d'azodicarboxylate de diisopropyle. Après 16 heures d'agitation à 20°C, le milieu réactionnel est concentré sous pression réduite, puis repris dans 20 mL de dichlorométhane et 20 ml d'une solution saturée en hydrogénocarbonate de sodium. La phase organique est séparée, lavée à l'eau, séchée sur sulfate de sodium puis concentrée sous pression réduite. Le produit obtenu est purifié par chromatographie sur colonne de silice en éluant avec un mélange de dichlorométhane, d'acétone et de méthanol.

On récupère ainsi 0,1 g de produit attendu.
MS: [MH]⁺ = 615
R.M.N. 1H (DMSO D₆), δ (ppm): 10,8 (s, 1H) ; 8,75 (m, 1H) ; 8,7 (m, 1H) ; 8,48 (m, 1H) ; 8,25 (s, 1H) ; 8,15 (dd, 1H) ; 8,05 (m, 1H) ; 7,82 (dd, 1H) ; 7,7 (ddd, 1H) ; 7,65 (s, 1H) ; 7,6 (dd, 1H) ; 7,35 (ddd, 1H) ; 7,08 (ddd, 1H) ; 6,95 (m, 2H) ; 6,5 (dd, 1H) ; 6,0 (s, 2H) ; 4,4 (m, 1H) ; 4,3 (m, 1H) ; 3,3 (m, 1H) ; 2,8 (m, 1H).

### 8.2 N-(6-Hydroxy-7,8-dihydro-6H-pyrrolo[2',1':2,3]imidazo[4,5-b]pyridin-3-yl)-5-trifluorométhyl-1-(3-fluorobenzyl)-1H-indole-2-carboxamide (Composé N°32)

A une solution de 0,065 g (0,07 mmole) de pyridine-2-carboxylate de 3-[[[1-(3-fluorobenzyl)-5-trifluorométhyl-1H-indol-2-yl]carbonyl]amino]-7,8-dihydro-6*H-*pyrrolo[2',1':2,3]imidazo[4,5-b]pyridin-6-yle préparé à l'exemple 8.1 dans 10 mL de chloroforme est ajoutée 0,022 g (0,7 mmole) de méthanol et 0,0127 g (0,07 mmole) d'acetate de cuivre. Après sept heures d'agitation à température ambiante, le mélange réactionnel est dilué avec 10 mL de dichlorométhane et 10 mL d'ammoniaque aqueux en agitant. La phase organique est séparée, lavée avec 10 mL d'eau, séchée sur sulfate de sodium et concentrée sous pression réduite. Le produit résultant est ensuite purifié par chromatographie successive sur colonne de silice puis sur colonne d'alumine neutre. On isole ainsi 0,012 g du produit attendu.
MS: [MH]⁺ : 510
R.M.N. 1 H (DMSO D₆), δ (ppm): 8,6 (s, 1H) ; 8,4 (s, 1H) ; 8,2 (s, 1H) ; 7,8 (d, 1H) ; 7,65 (s, 1H) ; 7,6 (d, 1H) ; 7,3 (m, 1H) ; 7,1 (m, 1H) ; 6,9 (m, 2H) ; 6,0 (pic élargi, 2H+ 1H) ; 5,15 (m, 1H) ; 4,3 (m, 1H) ; 4,1 (m, 1H) ; 2,95 (m, 1 H) ; 2,4 (m, 1H).
Pureté énantiomérique = 92% ; Temps de rétention = 8,7 mn

### Exemple 9 (Composé N°33)

### (-)-N-(6-Diméthylamino-7,8-dihydro-6H-pyrrolo[2',1':2,3]imidazo[4,5-b]pyridin-3-yl)-5-trifluorométhyl-1-(3-fluorobenzyl)-1H-indole-2-carboxamide

### 9.1 5-trifluorométhyl-1-(3-fluorobenzyl)-1H-indole-2-carboxamide

A une suspension, agitée à 20°C, de 8 g (23,72 mmoles) d'acide 5-trifluorométhyl-1-(3-fluorobenzyl)-1*H*-indole-2-carboxylique (WO2006072736), dans 150 mL de toluène sec, sont ajoutés 17,3 mL (237,2 mmoles) de chlorure de thionyle. Le mélange réactionnel est agité 2 heures à reflux puis est concentré sous pression réduite. Le produit résultant est repris par 25 mL da dichlorométhane et on verse cette solution, goutte à goutte, sur une solution de 9,32 mL d'ammoniaque, à 30% dans l'eau. Le mélange réactionnel est agité 14 heures à 20°C. Après ce temps, on recueille, par filtration, un solide que l'on triture dans 50 mL de pentane. On recueille après filtration et séchage sous pression réduite 5,87 g de produit attendu.
R.M.N. ¹H (DMSO-D₆), δ ppm : 8,28 (pic élargi, 1H) ; 8.13 (s, 1H) ; 7,77 (d, 1H) ; 7,6 (m, 2H) ; 7,41 (s, 1H) ; 7,32 (m, 1H) ; 7,05 (m, 1H) ; 6,9 (m, 2H) ; 6 (s, 2H).

### 9.2 (-)-N-(6-Diméthylamino-7,8-dihydro-6H-pyrrolo[2',1':2,3]imidazo[4,5-b]pyridin-3-yl)-5-trifluoromethyl-1-(3-fluorobenzyl)-1H-indole-2-carboxamide (Composé N°33)

On introduit successivement, sous atmosphère inerte dans un tube à pression, 0,4 g ( 1,19 mmole) de 5-trifluorométhyl-1-(3-fluorobenzyl)-1*H*-indole-2-carboxamide, préparé à l'exemple 9.1, 0,068 g (0,36 mmole) d'iodure de cuivre, 0,36 g (1,31 mmole) de composé IV-h 0,328 g (2,38 mmoles) de carbonate de potassium et de 0,044 g (0,39 mmole) de trans-1,2-diaminocyclohexane dans 10 mL de dioxane. Le tube est fermé puis agité à 130°C durant 12 heures. Après ce temps, le mélange est repris dans 60 mL d'un mélange 1:1 d'acétate d'éthyle et d'eau. La phase aqueuse est séparée puis lavée trois fois avec 20 mL d'acétate d'éthyle. Les phases organiques sont réunies, lavées deux fois avec 20 mL d'eau, puis séchées sur sulfate de sodium et concentrées sous pression réduite. Le produit résultant est ensuite purifié par chromatographie sur colonne de silice en éluant avec un mélange de dichlorométhane et de méthanol. On isole ainsi 87 mg du produit attendu.
PF = 203-205°C
MS: [MH]⁺ = 537
[α]_{D}²⁰ = -0,86°; c = 0,558 g/100 mL (méthanol)
R.M.N. ¹H (CDCl₃), δ (ppm): 9,19 (pic élargi, 1H) ; 8,37 (s, 1H) ; 8,15 (s, 1H) ; 7,8 (s, 1H) ; 7,32 (m, 2H) ; 7,16 (s, 1H) ; 7,08 (m, 1H) ; 6,78 (m, 2H) ; 6,64 (m, 1H) ; 5,72 (s, 2H) ; 4,05 (m, 2H) ; 3,91 (m, 1H) ; 2,74-2,4 (m, 2H) ; 2,21 (s, 6H).

### Exemple 10 (Composé N°34)

### N-(6-amino-7,8-dihydro-6H-pyrrolo[2',1':2,3]imidazo[4,5-b]pyridin-3-yl)-5-fluoro-1-(3-fluorobenzyl)-1H-indole-2-carboxamide

### 10.1 5-fluoro-1-(3-fluorobenzyl)-1H-indole-2-carboxamide

A une suspension, agitée à 20°C, de 2 g (6,96 mmoles) d'acide 5-fluoro-1-(3-fluorobenzyl)-1*H*-indole-2-carboxylique préparé selon le protocole décrit à l'étape 1.1, dans 80 ml de toluène sec, sont ajoutés 5,08 mL (69,62 mmoles) de chlorure de thionyle. Le mélange réactionnel est agité 2 heures à reflux puis est concentré sous pression réduite. Le produit résultant est repris par 10 mL de dichlorométhane et on verse cette solution, goutte à goutte, sur une solution de 9,12 mL (69,62 mmoles) d'ammoniaque à 30% dans l'eau. Le mélange réactionnel est agité 14 heures à 20°C. Après ce temps, on recueille, par filtration, un solide que l'on triture dans 50 mL d'éther diisopropylique. On recueille après filtration et séchage sous pression réduite 0,58 g de produit attendu.
R.M.N. ¹H (DMSO-D₆), δ ppm : 8,11 (pic élargi, 1H) ; 7,5 (m, 3H) ; 7,32 (m, 1H) ; 7,25 (s, 1H) ; 7,09 (m, 2H) ; 6,89 (m, 2H) ; 5.91 (s, 2H).

### 10.2 N-(6-amino-7,8-dihydro-6H-pyrrolo[2',1':2,3]imidazo[4,5-b]pyridin-3-yl)-5-fluoro-1-(3-fluorobenzyl)-1H-indole-2-carboxamide (Composé N°34)

On procède de façon similaire à l'exemple 9.2 à partir de 0,5 g (1,75 mmole) de 5-fluoro-1-(3-fluorobenzyl)-1*H*-indole-2-carboxamide décrit à l'étape précédente et de 0,659 g (1,92 mmole) de composé IV-e. On isole ainsi 208 mg du produit attendu.
PF = 218-220°C
MS: [MH]⁺ = 459
R.M.N. ¹H (CDCl₃), δ (ppm): 10,61 (s, 1H) ; 8,6 (s, 1H), 8,4 (m, 1H) ; 7,6 (m, 2H) ; 7.5 (s, 1H) ; 7,3 (m, 1H) ; 7,2 (m, 1H) ; 7,1 (m, 1H) ; 6,9 (m, 2H) ; 5,9 (s, 2H) ; 4,45 (m, 1 H) ; 4,25 (m, 1H) ; 4,0 (m, 1H) ; 2,9 (m, 1H) ; 2,3 (m, 1H).

### Exemple 1 (Composé N°35)

### N-(6-acétylamino-7,8-dihydro-6H-pyrrolo[2',1':2,3]imidazo[4,5-b]pyridin-3-yl)-5-fluoro-1-(3-fluorobenzyl)-1H-indole-2-carboxamide

A une solution, agitée à 40°C, de 0,05 g (0,11 mmole) de composé n°34, préparé à l'étape 10.2, dans 10 mL de dichlorométhane, est ajoutée une solution de 0,013 g (0,13 mmole) d'anhydride acétique dans 5 mL de dichlorométhane. Le mélange réactionnel est agité à 40°C puis est concentré sous prescion réduite. Le produit résultant est repris par 10 mL d'éther isopropylique, porter à ébullition puis filtré à chaud et séché sous pression réduite. On obtient ainsi 53 mg du produit attendu
PF = 282-283°C
MS: [MH]⁺= 501
R.M.N. ¹H (CDCl₃), δ (ppm) : 10,61 (s, 1H) ; 8,6 (m, 2H) ; 8,4 (s, 1H) ; 7,6 (m, 2H) ; 7,5 (s, 1H) ; 7,3 (s, 1H) ; 7,2 (m, 1H) ; 7,1 (m, 1H) ; 6,9 (m, 2H) ; 5,9 (s, 2H) ; 5,4 (q, 1H) ; 4,3 (m, 1H) ; 4,1 (m, 1H) ; 2,93 (m, 1H) ; 2,4 (m, 1H) ; 1,8 (s, 3H).

### Example 12 (Composé N°36)

### (-)-N-(8-Hydroxy-7,8-dihydro-6H-pyrrolo[2',1':2,3]imidazo[4,5-b]pyridin-3-yl)-5-trifluorométhyl-1-(3-fluorobenzyl)-1H-pyrrolo[2,3-b]-pyridine-2-carboxamide

### 12.1 5-Trifluorométhyl-1H-pyrrolo[2,3-b]pyridine-2-carboxylate d'éthyle

Dans un ballon de 100 mL, muni d'un agitateur magnétique, est introduit 0,3 g (1,3 mmole) d'acide 5-trifluorométhyl-1*H*-pyrrolo[2,3-*b*]pyridine-2-carboxylique (Angew Chem Int Ed 2004, 43(34), 4526-4528) et 50 mL d'éthanol. A cette solution, on ajoute 0,5 mL d'acide sulfurique concentré. Le mélange réactionnel est ensuite porté à reflux pendant 18 heures. La solution refroidie est concentrée à sec sous pression réduite. Le résidu est repris au dichlorométhane (100 mL), on lave la phase organique successivement avec une solution aqueuse de soude normale (30 mL), avec de l'eau (20 mL) puis avec une solution aqueuse saturée en chlorure de sodium. On la sèche sur sulfate de sodium puis on ta concentre sous pression réduite. On isole 0,29 g (1,12 mmole) du produit attendu sous forme d'une poudre jaune.
R.M.N. ¹H (DMSO D₆), δ (ppm) : 12,95 (s, NH) ; 8,8 (d, 1H) ; 8,6 (d, 1H) ; 7,3 (s, 1H) ; 4,4 (q, 2H) ; 1,35 (t, 3H).

### 12.2 5-Trifluorométhyl-1-(3-fluorobenzyl)-1H-pyrrolo[2,3-b]pyridine-2-carboxylate d'éthyle

A une solution de 0,3 g (1,16 mmole) de produit obtenu à l'étape 12.1, dans 20 mL de tétrahydrofurane sec, maintenue sous atmosphère inerte, sont ajoutés successivement sous agitation, 0,23 g (1,74 mmole) d'alcool 3-fluorobenzylique puis 0,46 g (1,74 mmole) de triphénylphosphine. On ajoute ensuite, goutte à goutte, 0,31 g (1,74 mmole) d'azodicarboxylate de diéthyle. Le mélange réactionnel est alors agité pendant 20 h à température ambiante puis concentré sous pression réduite. On purifie l'huile résultante par chromatographie sur colonne de gel de silice en éluant avec un mélange d'heptane et d'acétate d'éthyle. On isole 0,34 g (0,93 mmole) du produit attendu.
R.M.N. ¹H (DMSO D₆), δ (ppm) : 8,9 (d, 1H) ; 8,7 (d, 1H) ; 7,5 (s, 1H) ; 7,4-6,95 (m, 2H) ; 6,85 (m, 2H) ; 5,9 (s, 2H) ; 4,3 (q, 2H), 1,3 (t, 3H).

### 12.3 Acide 5-trifluorométhyl-1-(3-fluorobenzyl)-1H-pyrrolo[2,3-b]pyridine-2-carboxylique

Une solution de 3,15 g (8,6 mmoles) do 5-trifluorométhyl-1-(3-fluorobenzyl)-1*H-*pyrrolo[2,3-*b*]pyridine-2-carboxylate d'éthyle obtenu à l'étape 12.2 dans 100 mL d'éthanol et de 26 mL de soude 2N est agitée quatre heures à reflux. Après ce temps, le mélange réactionnel est concentré sous pression réduite puis repris dans 40 mL d'eau. Le milieu est acidifié à pH 3 par ajouts successifs d'acide chlorhydrique 1N. Le précipité est recueilli par filtration, lavé à l'eau puis séché sous pression réduite. On isole ainsi 2,9 g du produit attendu, sous la forme d'une poudre blanche, qui est utilisé tel quel dans l'étape suivante.
R.M.N. ¹H (DMSO D₆), δ (ppm): 13,5 (pic élargi, 1H) ; 8,81 (d, 1H) ; 8,63 (d, 1H) ; 7,47 (s, 1H) ; 7,3 (m, 1H) ; 7,1-6,8 (m, 3H) ; 5,94 (s, 2H).

### 12.4 (-)-N-(6-Hydroxy-7,8-dihydro-6H-pyrrolo[2',1':2,3]imidazo[4,5-b]pyridin-3-yl)-6-trifluorométhyl-1-(3-fluorobenzyl)-1H-pyrrolo[2,3-b]pyridine-2-carboxamide (Composé N°36)

On procède de façon analogue à la méthode décrite à l'étape 1.2, à partir de 0,3 g (0,89 mmole) d'acide 5-trifluorométhyl-1-(3-fluorobenzyl)-1*H*-pyrrolo[2,3-b]pyridine-2-carboxylique, préparé à l'étape 12.3, de 0,25 g (1,33 mmoles) du composé IV-c, de 0,19 g (0,98 mmole) de chlorhydrate de *N*-(3-diméthylaminopropyl)-*N-*éthylcarbodiimide et de 0,13 g (0,98 mmole) de *N*-1-hydroxybenzotriazole dans 10 mL de diméthylformamide. On obtient ainsi 0,350 g de produit attendu.
PF : 279-280°C
MS : [MH]+ : 511
[α]_{D}²⁰ :-3,367 ; c = 0,398 g/100 mL (DMSO)
RMN¹H (CDCl₃), δ (ppm) : : 8,8 (s, 1H) ; 8,7 (s, 1H) ; 8,5 (s, 1H) ; 8.4 (s, 1H) ; 7,6 (s, 1H) ; 7,3 (m, 1H) ; 7,1 (m, 3H) ; 6,0 (pic élargi, 2H+ 1H) ; 5,2 (m, 1H) ; 4,25 (m, 1H) ; 4,1 (m, 1H) ; 3,0 (m, 1H) ; 2,4 (m, 1H).

### Example 13 (Composé N°28)

### (-)-N-(6-Hydroxy-7,8-dihydro-6H-pyrrolo[2',1':2,3]imidazo[4,5-b]pyridin-3-yl)-6-trifluorométhyl-1-(3-fluorobenzyl)-1H-indole-2-carboxamide

On procède de façon analogue à la méthode décrite à l'étape 1.2, à partir de 0,3 g (0,89 mmole) d'acide 6-trifluorométhyl-1-(3-fluorobenzyl)-1*H*-indole-2-carboxylique (WO2006072736), de 0,25 g (1,33 mmoles) du composé IV-c, de 0,19 g (0,98 mmole) de chlorhydrate de *N*-(3-diméthylaminopropyl)-*N'*-éthylcarbodiimide et de 0,13 g (0,98 mmole) de *N*-1-hydroxybenzotriazole dans 10 mL de diméthylformamide. On obtient 0,30 g de produit attendu.
PF = 269-271 °C
MS: [MH]⁺ = 510
[α]_{D}²⁰ = -2,614°, c = 0,306 g/100 mL ; DMSO
R.M.N. ¹H (CDCl₃), 5 (ppm) : 8,6 (d, 1H) ; 8,4 (d, 1H) ; 8,1 (m, 2H) ; 7,6 (s, 1H) ; 7,5 (d, 1H) ; 7,35 (m, 1H) ; 7.1 (m, 3H) ; 6,9 (pic élargi, 2H+ 1H) ; 6,0 (s, 2H) ; 5,2 (m, 1H); 4,3 (m, 1H) ; 4,1 (m, 1H) ; 2,9 (m, 1H) ; 2,4 (m, 1H).

Selon une méthode analogue aux méthodes décrites dans les exemples ci-dessus, on peut préparer les composés n°37 et 38.

### Example 14 (Composé N°37)

### (+)-N-(6-Fluoro-7,8-dihydro-6H-pyrrolo[2',1':2,3]imidazo[4,5-b]pyridin-3-yl)-6-trifluorométhyl-1-(3-fluorobenzyl)-1H-indole-2-carboxamide

[α]_{D}²⁰ =+33,10°, c = 0,113 g/100 mL ; MeOH
R.M.N. ¹H (DMSO D₆), δ (ppm): 10.8 (s, 1H) ; 8,7 (s, 1H) ; 8,5 (s, 1H) ; 8,1 (s, 1H) ; 8 (d, 1H) ; 7,6 (s, 1H) ; 7,5 (d, 1H) ; 7,35 (m, 1H) ; 7,1 (m, 1H) ; 6,9 (m, 2H) ; 6,25 - 6,1 (m, 1H) ; 6 (s, 2H) ; 4,4 (m, 1H) ; 4,3 (m, 1H) ; 3,15 (m, 1H) ; 2,8 (m, 1H).

### Exemple 15 (Composé N°38)

### N-(6-Amino-7,8-dihydro-6H-pyrrolo[2',1':2,3]imidazo[4,5-b]pyridin-3-yl)-5-trifluorométhyl-1-(3-fluorobenzyl)-1H-indole-2-carboxamide

[α]_{D}²⁰: non déterminé
R.M.N. ¹H (CDCl₃), δ (ppm): 9,1 (s, 1H) ; 8,3 (d, 1H) ; 8,1 (s, 1H) ; 7,8 (s, 1H) ; 7,3 (m, 2H) ; 7,15 (m, 1H) ; 6,7 (m, 4H) ; 5,8 (s, 2H) ; 4,4 (t, 1H) ; 4,2 (m, 1H) ; 3,95 (m, 1H) ; 2,9 (m, 1H) ; 2,3 (m, 1H) ; 2.0 (pic élargi, 2H).

Le tableau 2 qui suit illustre les structures chimiques et les propriétés physiques de quelques exemples de composés selon l'invention.

Dans ce tableau:
- la colonne « Configuration » indique, quand il y a liau, la valeur du pouvoir rotatoire du composé en degré, mesuré à la température de 20°C, ainsi que le solvant employé et la concentration en g/100 mL de l'échantillon ; ou bien la pureté énantiomérique mesurée par chromatographie phase chirale. « (+/-) » indique que le composé est un mélange racémique.
- la colonne « PF (°C) » renseigne tes points de fusion des produits on degrés Celsius (°C) ;
- les composés sont sous forme de base libre ;
- « Ac » correspond à un groupe acétyle; « OH » correspond à un groupe hydroxy ; « Ph » correspond à un groupe phényle.

**Tableau 2**

| | | | | | | |
|---|---|---|---|---|---|---|
| 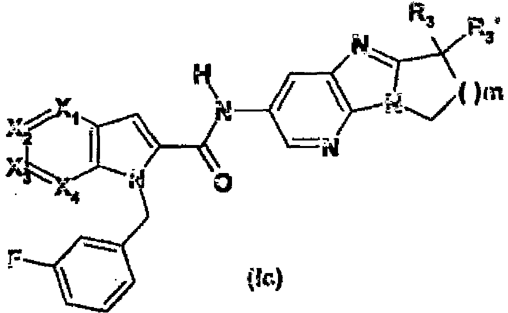 | | | | | | |

| **N°** | **X₁, X₂, X₃, X₄** | **R₃** | **R'₃** | **m** | **Configuration [α]_{D}²⁰ (°); g/100mL; solvant** | **PF (°C)** |
|---|---|---|---|---|---|---|
| **1** | CH, C-F, CH, CH | H | H | 1 | - | 227-229 |
| **2** | CH, C-F, CH, CH | OAc | H | 1 | (+/-) | 139 - 141 |
| **3** | CH, C-F, CH, CH | OH | H | 1 | (+/-) | 288 - 290 |
| **4** | C-F, CH, CH, CH | H | H | 1 | - | 265 - 266 |
| **5** | CH, CH, CH, C-F | H | H | 1 | - | 237 - 239 |
| **6** | CH, CH, C-F, CH | H | H | 1 | - | 235 - 236 |
| **7** | CH, CH, C-CF₃, CH | H | H | 1 | - | 261 - 282 |
| **8** | CH, C-CF₃, CH, CH | H | H | 1 | - | 223 - 225 |
| **9** | C-Cl, CH, CH, CH | H | H | 1 | - | 302 - 303 |
| **10** | CH, CH, C-Cl, CH | H | H | 1 | - | 252 - 253 |
| **11** | CH, C-Cl, CH, CH | H | H | 1 | - | 263 - 264 |
| **12** | CH, CH, CH, CH | H | H | 1 | - | 243 - 244 |
| **13** | C-CH₃, CH, CH, CH | H | H | 1 | - | 303 - 304 |
| **14** | CH, CH, C-CH₃, CH | H | H | 1 | - | 182 - 183 |
| **15** | CH, C-CH₃, CH, CH | H | H | 1 | - | 237 - 238 |
| **16** | CH, CH, C-CH₂CH₃, CH | H | H | 1 | - | 224 - 226 |
| **17** | CH, CH, C-CH(CH₃)₂, CH | H | H | 1 | - | 275 - 276 |
| **18** | CH, C-C(CF₃)₃, CH, CH | H | H | 1 | - | 234 - 236 |
| **19** | CH, CH, C-C(CH₃)₃, CH | H | H | 1 | - | 244 - 245 |
| **20** | C-CH₃, CH, CH, C-CH₃ | H | H | 1 | - | 286 - 288 |
| **21** | CH, C-SO₂-CH₃,CH, CH | H | H | 1 | - | 275 - 277 |
| **22** | CH, CH, C-SCH₃, CH | H | H | 1 | - | 222 - 223 |
| **23** | CH, CH, C-CF₃, N | H | H | 1 | - | 272 - 273 |
| **24** | CH, C-CF₃, CH, N | H | H | 1 | - | 247 - 249 |
| **25** | CH, C-F, CH, N | H | H | 1 | - | 242 - 243 |
| **26** | CH, C-CF₃, CH, CH | OH | H | 1 | Pureté énantiomérique = 94% ; Tr = 14,5 mn * | 267 - 268 |
| **27** | CH, C-CF₃, CH, CH | H | H | 2 | - | 262 - 263 |
| **28** | CH, CH, C-CF₃, CH | OH | H | 1 | -2,614°, c = 0,306 ; DMSO | 269 - 271 |
| **29** | CH, C-CF₃, CH, CH | OH | H | 1 | (+/-) | 254 - 255 |
| **30** | CH, C-CF₃, CH, CH | Ph | H | 1 | (+/-) | 129 - 130 |
| **31** | CH, C-CF₃, CH, CH | Me | Me | 1 | - | 212 - 213 |
| **32** | CH, C-CF₃, CH, CH | OH | H | 1 | Pureté énantiomérique = 92%, Tr = 8,7 mn * | 510 * |
| **33** | CH, C-CF₃, CH, CH | N(CH₃)₂ | H | 1 | -0,86, c = 0,558 ; Méthanol | 203 - 205 |
| **34** | CH, C-F, CH, CH | NH₂ | H | 1 | (+/-) | 218 - 220 |
| **35** | CH, C-F, CH, CH | NHAc | H | 1 | (+/-) | 282 - 283 |
| **36** | CH, C-CF₃, CH, N | OH | H | 1 | -3,367 ; c = 0,398 DMSO | 279 - 280 |
| **37** | CH, CH, C-CF₃, CH | F | H | 1 | +33,10°, c = 0,113 ; MeOH | 512 ** |
| **38** | CH, C-CF₃, CH, CH | NH₂ | H | 1 | non déterminé | 509 ** |

| | | | | | | |
|---|---|---|---|---|---|---|
| ** Valeur [MH]+ mesurée par spectrométrie de masse * Les temps de rétention (Tr) ont été mesurés par HPLC chirale sur une colonne Chiralpak AD-H (250*4,6 mm ; 3,5 µM), en utilisant une solution d'éthanol et de Diéthylamine 1000 / 1 comme eluant, à température ambiante, avec un débit de 0,8 mL/mn, détection à 220 nM, en injectant 10 µL d'une solution dans l'éthanol. | | | | | | |

Les composés selon l'invention ont fait l'objet d'essais pharmacologiques *in vitro* et *in vivo* qui ont mis en évidence leur intérêt comme substances à activités thérapeutiques. Ces composés présentent une activité antagoniste ou agoniste vis-à-vis des récepteurs TRPV1 (ou VR1).

### Test d'inhibition du courant induit par la capsaïcine sur les DRG de rat

- Culture primaire de cellules de ganglions de racine dorsale (DRG) de rat :
Les neurones du DRG expriment naturellement le récepteur TRPV1.
Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 µg/mL gentamicine et 50 ng/mL de NGF, puis déposées sur des lamelles de verre recouvertes de laminine (0,25 x 10⁶ cellules par lamelle) qui sont ensuite placées dans des boîtes 12 puits Coming. Les cellules sont incubées à 37°C en atmosphère humidifiée contenant 5% de CO₂ et 95% d'air. De la cytosine β-D-arabinoside (1 µM) est ajoutée 48 h après la mise en culture, pour prévenir le développement des cellules non neuronales. Les lamelles sont transférées dans les chambres expérimentales pour les études de patch-clamp après 7-10 jours de culture.
- Electrophysiologie :
Les chambres de mesure (volume 800 µl) contenant la préparation cellulaire sont placées sur la platine d'un microscope inversé (Olympus IMT2) équipé d'optiques Hoffman (Modulation Contrast, New York) et observées au grossissement de 400X.
Les chambres sont continuellement perfusées par gravité (2,5 mL/min) à l'aide d'un distributeur de solutions acceptant 8 entrées et dont la sortie unique, constituée par un tube de polyéthylène (ouverture 500µm) est placée à moins de 3 mm de la cellule étudiée. La configuration "cellule entière" de la technique de patch-clamp a été utilisée.
Les pipettes en verre borosilicaté (résistance 5-10 MOhme) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1D (Axon instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution de capsaïcine 300 nM provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés de l'invention à tester sont appliqués seuls à une concentration donnée (concentration de 10 nM ou de 1 nM) pendant une durée de 4 à 5 minutes, au cours desquelles plusieurs tests « capsaïcine + composé » sont réalisés (obtention de l'inhibition maximale). Les résultats sont exprimés en pourcentage d'inhibition de la réponse capsaïcine contrôle.
Dans le cas des composés antagonistes VR1, les pourcentages d'inhibition de la réponse capsaïcine (1µM) sont compris entre 20% et 100% pour les composés les plus actifs de l'invention testés à des concentrations de 0,1 à 10 nM. Ce sont donc des antagonistes efficaces des récepteurs de type TRPV1. Le tableau 3 donne un exemple de pourcentage d'inhibition obtenu avec les composés de invention.

**Tableau 3**

| **N° composé** | **% inhibition en patch DRG** |
|---|---|
| **1** | 69 % (10 nM) |

### Douleur induite par l'administration intraplantaire de capsaicine chez la souris.

L'injection intraplantaire de capsaicine chez la souris produit rapidement un comportement nociceptif de courte durée, qui se traduit par des léchages, des mordillements et un fléchissement de la patte administrée. Ces réponses nociceptives sont probablement liées à activation des récepteurs TRPV1 locaux par la capsaicine.

### Méthodologie :

La (E)-capsaicine est initialement diluée à 3 mg/ml dans du DMSO, puis diluée à nouveau pour son utilisation finale à 1,5 µg / 20µl dans du sérum physiologique.

L'administration de solvant n'a aucun effet sur le comportement de la souris. La capsaicine est injectée dans une des pattes arrière de l'animal, au niveau de la face supérieure.

Les composés à tester sont administrés par voie orale 120 minutes avant l'injection de capsaicine. Deux heures après l'administration des composés, les souris sont placées dans un bêcher en verre. Le comportement nociceptif des animaux est alors immédiatement évalué par l'expérimentateur et la durée des manifestations comportementales induites par la capsaicine est chronométrée pendant une période de 2 minutes (léchages et mordillements, fléchissement total ou partiel de la patte injectée).

Pour chaque composé, est déterminée une inhibition correspondant à la moyenne des réponses nociceptives induites par la capsaicine, en réponse à une dose de produit étudié (exprimée en mg/kg) administrée par voie orale sur un échantillon d'un nombre déterminée de souris (n).

Le tableau 4 donne un exemple de pourcentage d'inhibition obtenu avec les composés de l'invention.

**Tableau 4**

| **N° composé** | **Dose** | **n** | **% inhibition des réponses nociceptives induites par la capsaicine** |
|---|---|---|---|
| **29** | 30 mg/kg | 10 | 63 % (+/- 11%) |

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Les composés de l'invention peuvent être utiles pour prévenir ou traiter les pathologies dans lesquelles les récepteurs de type TRPV1 sont impliqués.

Ainsi, l'invention a pour objet des médicaments qui comprennent au moins un composé de formule (I) selon l'invention, ou un sel pharmaceutiquement acceptable, ou encore un hydrate ou un solvate dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou iatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la névralgie post-herpétique, de la douleur musculaire, de la compression nerveuse (centrale et/ou périphérique), des traumatismes de la moelle et/ou du cervesu, de l'ischémie (de la moelle et/ou du cerveau), de la neurodégénérescence, des accidents vasculaires hémorragiques (de la moelle et/ou du cerveau), de la douleur post-stroke.

Les composés de l'invention peuvent également être utilisés pour prévenir et/ou traiter les désordres métaboliques tels que le diabète et l'obésité.

Les composés de l'invention peuvent également être utilisés pour prévenir et/ou traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

Les composés de l'invention peuvent être utiles pour prévenir et/ou traiter les désordres gynécologiques comme la vulvodynie, les douleurs liées aux salpingites, aux dysménorrhées.

On peut également utiliser ces produits pour prévenir et/ou traiter les désordres gastro-intestinaux tels que le désordre du réflexe gastro-esophagique, l'ulcère de l'estomac, l'ulcère du duodénum, la dyspepsie fonctionnelle, la colite, l'IBS, la maladie de Crohn, la pancréatite, l'oesophagite, la colique hépatique.

De même, les produits de la présente invention peuvent être utiles dans la prévention et/ou le traitement des désordres respiratoires tels que l'asthme, la toux, la maladie pulmonaire obstructive chronique (COPD), la bronchoconstriction et les désordres inflammatoires.

Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour traiter la dépression. Les composés de l'invention peuvent aussi être utilisés pour traiter les maladies du système nerveux central telles que la sclérose en plaques, la maladie de Parkinson, et la maladie de Huntington.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Les compositions pharmaceutiques de la présente invention peuvent être administrées par voie orale, sublinguale, sous-cutanée, intramusculaire, intra-veineuse, topique, locale, intratrachéale, intranasale, transdermique ou rectale. Ces compositions peuvent être administrées sous forme unitaire, en mélange avec des excipients pharmaceutiques classiques. Elles sont destinées à être administrées aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou duras, les poudras, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Les composés de l'invention peuvent également être utilisés pour la préparation de médicaments, notamment pour la préparation d'un médicament destiné à prévenir ou à traiter les pathologies dans lesquelles les récepteurs de type TRPV1 sont impliqués, telles que mentionnées précédemment.

La présente invention, selon un autre de ses aspects, décrit également une méthode de traitement des pathologies indiquées ci-dessus, qui comprend l'administration, à un patient, d'une dose efficace d'au moins un composé selon l'invention, ou un de ses sels pharmaceutiquement acceptables ou hydrates ou solvates.

## Revendications

1. Composé répondant à la formule (I) 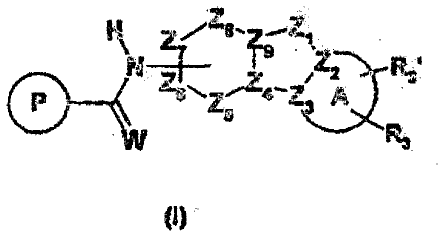 dans laquelle :
le groupe 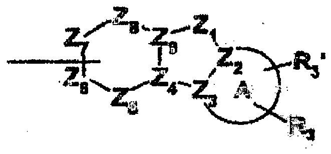 est choisi parmi : 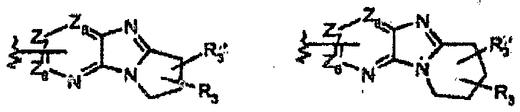
Z₆, Z₇ et Z₈ représentent, indépendamment l'un de l'autre, un atome de carbone lié à l'atome d'azote de l'amide de formule (I), ou un groupe C-R₂;
R₂ représente un atome d'hydrogène :
R₃ et R₃' sont portés par un atome de carbone et représentent, indépendamment l'un de l'autre, un atome d'hydrogène, un atome d'halogène, un groupe C₁-C₈-alkyle, hydroxyle, C₁-C₈-alkyle-C(O)-O-, NR₄R₅, NR₆C(O)R₇ ou aryle ;
R₄ et R₅, représentent, indépendamment l'un de l'autre, un atome d'hydrogène ou un groupe C₁-C₆-alkyle ;
R₆ et R₇ représentent, indépendamment l'un de l'autre, un atome d'hydrogène ou un groupe C₁-C₆-alkyle.
W représente un atome d'oxygène ;
P représente un groupe indolyle ou pyrrolo[2,3-b]pyridinyle tel que représenté ci dessous, lié en position C-2 au carbonyle de l'amide de formule (I) : 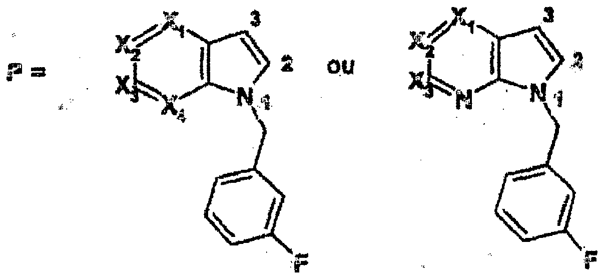
X₁, X₂, X₃, X₄ représentent, indépendamment l'un de l'autre, un groupe C-R₁ ;
R₁ est choisi parmi un atome d'hydrogène, un atome d'halogène, C₁-C₆-alkyle, C₁-C₆-fluoroalkyle, C₁-C₆-thioalkyle, C₁-C₆-alkyle-S(O)₂- ;
le ou les atomes d'azote pouvant éventuellement être sous forme oxydée (N-oxyde) à l'état de base ou de sel d'addition à un acide, ainsi qu'à l'état d'hydrate ou de solvate.

2. Procédé de préparation d'un composé de formule (I) selon la revendication 1, **caractérisé en ce que** l'on fait réagir un composé de formule générale (II) : 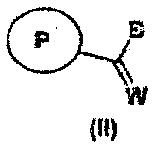 dans laquelle P et W sont tels que définis dans la formule générale (I) selon la revendication 1,
avec un composé de formule générale (III), 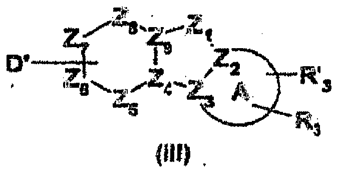 dans laquelle A, Z₁, Z₂, Z₃, Z₄, Z₅, Z₆, Z₇, Z₈, Z₉, R₃ et R₃' sont tels que définis dans la formule générale (I) selon la revendication 1,
- quand B représente un groupe C₁-C₆-alcoxy, C₃-C₇-cycloalkyloxy, C₃-C₇-cycloalkyl-C₁-C₃-alkylénoxy, aryle-C₁-C₃-alkylénoxy, avec un amidure du composé de formule générale (III), au reflux d'un solvant, l'amidure d'aluminium du composé de formule générale (III) étant préparé par action préalable du triméthylaluminium sur le composé de formule générale (III), dans laquelle D' représente un groupe NH₂ ;
- quand B représente un groupe hydroxyle et W représente un atome d'oxygène,
soit par transformation de la fonction acide carboxylique en halogénure d'acide puis par réaction du composé de formule générale (II), dans laquelle B représente un atome de chlore et W représente un atome d'oxygène, avec le composé de formule générale (III), dans laquelle D' représente un groupe NH₂, en présence d'une base ;
soit par couplage du composé de formule générale (II) avec les composés de formule générale (III), dans laquelle D' représente un groupe NH₂, en présence d'un agent de couplage et d'une base dans un solvant ;
- quand B représente un groupe NH₂ et W représente un atome d'oxygène, par réaction du composé de formule générale (II) avec le composé de formule générale (III), dans laquelle D' correspond à un groupe partant, en présence d'un sel de cuivre en quantité catalytique, en présence d'une quantité catalytique d'un ligand du cuivre, le tout en présence d'une base dans un solvant.

3. Composé de formule (IV) 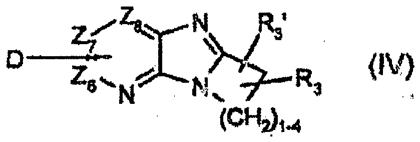 dans laquelle
Z₆, Z₇ et Z₈ représentent, indépendamment l'un de l'autre, un atome de carbone ou un groupe C-R₂ ; l'un des Z₆, Z₇ et Z₈ correspond à un atome de carbone et porte le groupe D ;
D représente un atome d'halogène ou un groupe NH₂ ;
R₂ représente un atome d'hydrogène ;
R₃ et R₃', représentent, indépendamment l'un de l'autre, un atome d'hydrogène, un atome d'halogène, un groupe C₁-C₆-alkyle, C₃-C₇-cycloakyle, C₃-C₇-cycloalkyl-C₁-C₃-alkylène, C₁-C₆-fluoroalkyle, hydroxyle, thiol, C₁-C₆-alcoxy, C₃-C₇-cycloalkyloxy, C₃-C₇-cycloalkyl-C₁-C₃-alkylénoxy, aryle-C(O)-O-, C₁-C₆-alkyle-C(O)-O-, C₃-C₇-cycloalkyl-C(O)-O-, C₃-C₇-cycloalkyl-C₁-C₃-alkylène-C(O)-O, C₁-C₆-fluoroalkyle-C(O)-O-, oxo, thio, NR₄R₅, NR₆C(O)R₇, hétéroaryle ou aryle ; les groupes hétéroaryle et aryle étant éventuellement substitués par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₈-fluoroalkyle, C₁-C₆-alcoxy, C₃-C₇-cycloalkyloxy, C₃-C₇-cycloalkyl-C₁-C₃-alkylénoxy, C₁-C₆-fluoroalcoxy, nitro ou cyano ;
ou bien R₃ et R₃' forment ensemble, avec l'atome de carbone qui les porte, un groupe C₃-C₇-cycloalk-1,1-diyle.
R₄ et R₅, représentent, indépendamment l'un de l'autre, un atome d'hydrogène ou un groupe C₁-C₈-alkyle, C₃-C₇-cycloalkyle, C₃-C₇-cycloalkyl-C₁-C₃-alkylène, aryle-C₁-C₆-alkylène ou aryle, ou R₄ et R₅ forment ensemble, avec l'atome d'azote qui les porte, un groupe azétidine, pyrrolidine, pipéridine, azépine, morpholine, thiomorpholine, pipérazine, homopipérazine, le groupe NR₄R₅ étant éventuellement substitué par un groupe C₁-C₆-alkyle, C₃-C₇-cycloalkyle, C₃-C₇-cycloalkyl-C₁-C₃-alkylène, aryle-C₁-C₆-alkylène, aryle, hétéroaryle, aryle-S(O)₂-, C₁-C₆-alkyle-S(O)₂-, C₁-C₆-fluoroalkyle-S(O)₂, C₃-C₇-cycloalkyl-S(O)₂, C₃-C₇-cycloalkyl-C₁-C₃-alkylène-S(O)₂-, aryle-C(O)-, C₁-C₆-alkyle-C(O)-, C₃-C₇-cycloalkyl-C(O)-, C₃-C₇-cycloalkyl-C₁-C₃-alkylène-C(O)-, C₁-C₆-fluoroalkyle-C(O)-, hydroxy, C₁-C₆-alkyloxy, C₃-C₇-cycloalkyloxy, C₃-C₇-cycloalkyl-C₁-C₃-alkylénoxy, C₁-C₆-fluoroalkyle, aryloxy-C₁-C₆-alkylène, aryloxy, hétéroaryloxy-C₁-C₆-alkylène, hétéroaryloxy ;
R₆ et R₇ représentent, indépendamment l'un de l'autre, un atome d'hydrogène, un groupe C₁-C₆-alkyle, C₃-C₇-cycloalkyle, C₃-C₇-cycloalkyl-C₁-C₃-alkylène, aryle-C₁-C₃-alkylène au aryle; le groupe aryle étant éventuellement substitué par un ou plusieurs substituants choisis parmi un atome d'halogène, un groupe C₁-C₆-alkyle, C₃-C₇-cycloalkyle, C₃-C₇-cycloalkyl-C₁-C₃-alkylène, C₁-C₆-fluoroalkyle, C₁-C₆-alcoxy, C₃-C₇-cycloalkyloxy, C₃-C₇-cycloalkyl-C₁-C₃-alkylénoxy, C₁-C₈-fluoroalcoxy, nitro ou cyano ;
ou R₆ et R₇ forment ensemble un lactame de 4 à 7 chaînons comprenant l'atome d'azote et le groupe C(O) qui les portent.

4. Médicament, **caractérisé en ce qu'**il comprend un composé de formule (1) selon la revendication 1, ou un sel d'addition de ce composé à un acide pharmaceutiquement acceptable, ou encore un hydrate ou un solvate du composé de formule (I).

5. Composition pharmaceutique, **caractérisée en ce qu'**elle comprend un composé de formule (I) selon la revendication 1, ou un sel pharmaceutiquement acceptable, un hydrate ou un solvate de ce composé, ainsi qu'au moins un excipient pharmaceutiquement acceptable.

6. Utilisation d'un composé de formule (I) selon la revendication 1 pour la préparation d'un médicament destiné à la prévention et au traitement des pathologies dans lesquelles les récepteurs de type TRPV1 sont impliqués, lesdites pathologies étant choisies parmi la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la dépression et les maladies du système nerveux central.

## Patentansprüche

1. Verbindung der Formel (I) 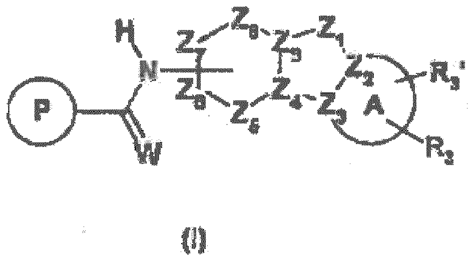 in der:
die Gruppe 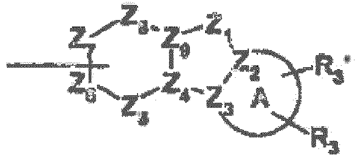 der Reihe 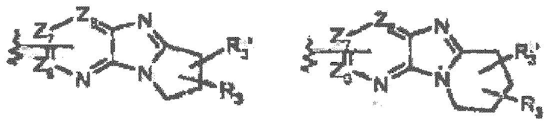 ausgewählt ist,
Z₆, Z₇ und Z₈ unabhängig voneinander ein Kohlenstoffatom, das an das Stickstoffatom des Amids der Formel (I) gebunden ist, oder eine C-R₂-Gruppe bedeuten;
R₂ ein Wasserstoffatom bedeutet;
R₃ und R₃' von einem Kohlenstoffatom getragen werden und unabhängig voneinander ein Wasserstoffatom, ein Halogenatom, eine C₁-C₆-Alkyl-, Hydroxyl-, C₁-C₆-Alkyl-C(O)-O-, NR₄R₅-, NR₆C(O)R₇- oder Arylgruppe bedeuten;
R₄ und R₅ unabhängig voreinander ein Wasserstoffatom oder eine C₁-C₆-Alkylgruppe bedeuten;
R₆ und R₇ unabhängig voneinander ein Wasserstoffatom oder eine C₁-C₆-Alkylgruppe bedeuten;
W ein Sauerstoffatom bedeutet;
P eine Indolyl- oder Pyrrolo[2,3-b]-pyridinylgruppe wie unten angegeben bedeutet, die in C-2-Stellung an das
Carbonyl des Amids der Formel (I) gebunden ist; 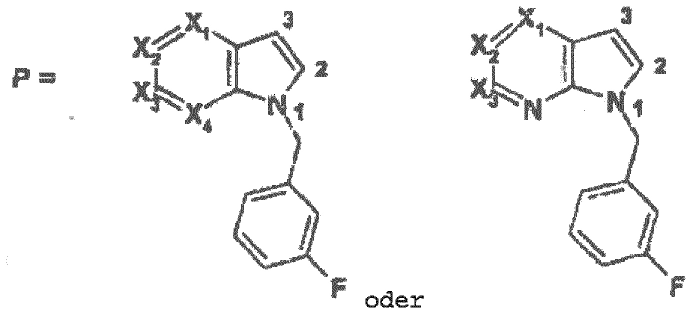
X₁, X₂, X₃, X₄ unabhängig voneinander eine C-R₁-Gruppe bedeuten;
R₁ aus der Reihe Wasserstoffatom, Halogenatom, C₁-C₆-Alkyl, C₁-C₆-Fluoralkyl, C₁-C₆-Thioalkyl, C₁-C₆-AlkylS(O)₂- ausgewählt ist;
wobei das Stickstoffatom bzw. die Stickstoffatome gegebenenfalls in oxidierter Form (N-Oxid) vorliegen kann/können,
als Base oder Säureadditionssalz sowie als Hydrat oder Solvat.

2. Verfahren zur Herstellung einer Werbindung der Formel (I) nach Anspruch 1, **dadurch gekennzeichnet, dass** man eine Verbindung der allgemeinen Formel (II) 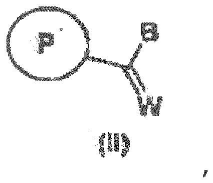 in der P und W wie in der allgemeinen Formel (I) nach Anspruch 1 definiert sind,
mit einer Verbindung der allgemeinen Formel (III), 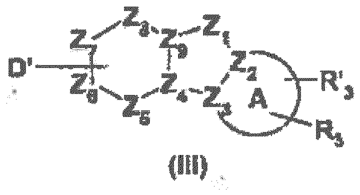 in der A, Z₁, Z₂, Z₃, Z₄, Z₅, Z₆, Z₇, Z₈, Z₉, R₃ und R₃' wie in der allgemeinen Formel (I) nach Anspruch 1 definiert sind, umsetzt; und zwar,
- wenn B eine C₁-C₆-Alkoxy-, C₃-C₇-Cycloalkyloxy-, C₃-C₇-Cycloalkyl-C₁-C₃-alkenyloxy-, Aryl-C₁-C₃-alkylenoxygruppe bedeutet, unter Lösungsmittelrückfluss mit einem Amid der Verbindung der allgemeinen Formel (III), wobei das Aluminiumamid der Verbindung der allgemeinen Formel (III) durch vorheriges Einwirken von Trimethylaluminium auf die Verbindung der allgemeinen Formel (III), in der D' eine NH₂-Gruppe bedeutet, hergestellt worden ist;
- wenn B eine Hydroxylgruppe bedeutet und W ein Sauerstoffatom bedeutet,
entweder durch Umwandeln der Carbonsäurefunktion in ein Säurehalogenid und anschließendes Umsetzen der Verbindung der allgemeinen Formel (II), in der B ein Chloratom bedeutet und W ein Sauerstoffatom bedeutet, mit der Verbindung der allgemeinen Formel (III), in der D' eine NH₂-Gruppe bedeutet, in Gegenwart einer Base;
oder durch Kuppeln der Verbindung der allgemeinen Formel (II) mit den Verbindungen der allgemeinen Formel (III), in der D' eine NH₂-Gruppe bedeutet, in Gegenwart eines Kupplungsmittels und einer Base in einem Lösungsmittel;
- wenn B eine NH₂-Gruppe bedeutet und W ein Sauerstoffatom bedeutet, durch Umsetzen der Verbindung der allgemeinen Formel (II) mit der Verbindung der allgemeinen Formel (III), in der D' eine Abgangsgruppe bedeutet, in Gegenwart einer katalytischen Menge eines Kupfersalzes, in Gegenwart einer katalytischem Menge eines Kupferliganden, und zwar in Gegenwert einer Base in einem Lösungsmittel.

3. Verbindung der Formel (IV), 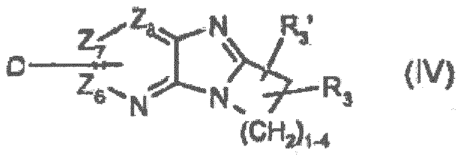 in der
Z₆, Z₇ und Z₈ unabhängig voneinander ein Kohlenstoffatom oder eine C-R₂-Gruppe bedeuten; wobei eines von Z₆, Z₇ und Z₈ einem Kohlenstoffatom entspricht und die Gruppe D trägt;
D ein Halogenatom oder eine NH₂-Gruppe bedeutet;
R₂ ein Wasserstoffatom bedeutet;
R₃ und R₃' unabhängig voreinander ein Wasserstoffatom, ein Halogenatom, eine C₁-C₆-Alkyl-, C₃-C₇-Cycloalkyl-, C₃-C₇-Cycloalkyl-C₁-C₃-alkylen-, C₁-C₆-Fluoralkyl-, Hydroxyl-, Thiol-, C₁-C₆-Alkoxy-, C₃-C₇-Cycloalkyloxy-, C₃-C₇-Cycloalkyl-C₁-C₃-alkylenoxy-, Aryl-C(O)-O-, C₁-C₆-Alkyl-C(O)-O-, C₃-C₇-Cycloalkyl-C(O)-O-, C₃-C₇-Cycloalkyl-C₁-C₃-alkylen-C(O)-O-, C₁-C₆-Fluoralkyl-C(O)-O-, Oxo-, Thio-, NR₄R₅-, NR₆C(O)R₇-, Heteroaryl- oder Arylgruppe bedeuten, wobei die Heteroarylgruppe und die Arylgruppe gegebenenfalls durch einen oder mehrere Substitutenten, substituiert sind, die aus einem Halogenatom, einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro- oder Cyanogruppe ausgewählt sind;
oder R₃ und R₃' gemeinsam mit dem Kohlenstoffatom, von dem sie betragen werden, eine C₃-C₇-Cycloalk-1,1-diylgruppe bilden,
R₄ und R₅ unabhängig voneinander ein Wasserstoffatom oder eine C₁-C₆-Alkyl-, C₃-C₇-Cycloalkyl-, C₃-C₇-Cycloalkyl-C₁-C₃-alkylen-, Aryl-C₁-C₅-alkylen- oder Arylgruppe bedeuten, oder R₄ und R₅ gemeinsam mit dem Stickstoffatom, von dem sie betragen werden, eine Azetidin-, Pyrrolidin-, Piperidin-, Azepin-, Morpholin-, Thiomorpholin-, Piperazin-, Homopiperazingruppe bilden, wobei die NR₄R₅-Gruppe gegebenenfalls durch eine C₁-C₆-Alkyl-, C₃-C₇-Cycloalkyl-, C₃-C₇-Cycloalkyl-C₁-C₃-alkylen-, Aryl-C₁-C₆-alkylen-, Aryl-, Heteroaryl-, Aryl-S(O)₂-, C₁-C₆-Alkyl-S(O)₂-, C₁-C₆-Fluoralkylen-S(O)₂-, C₃-C₇-Cycloalkyl-S(O)₂-, C₃-C₇-Cycloalkyl-C₁-C₃-alkylen-S(O)₂-, Aryl-C(O)-, C₁-C₆-Alkyl-C(O)-, C₃-C₇-Cycloalkyl-C(O)-, C₃-C₇-Cycloalkyl-C₁-C₃-alkylen-C(O)-, C₁-C₆-Fluoralkyl-C(O)-, Hydroxy-, C₁-C₆-Alkyloxy-, C₃-C₇-Cycloalkyloxy-, C₃-C₇-Cycloalkyl-C₁-C₃-alkylenoxy-, C₁-C₆-Fluoralkyl-, Aryloxy-C₁-C₆-alkylen-, Aryloxy-, Heteroaryloxy-C₁-C₆-alkylen- oder Heteroaryloxygruppe substituiert ist;
R₆ und R₇ unabhängig voneinander ein Wasserstoffatom, eine C₁-C₆-Alkyl-, C₃-C₇-Cycloalkyl-, C₃-C₇-Cycloalkyl-C₁-C₃-alkylen-, Aryl-C₁-C₆-alkylen- oder Arylgruppe bedeuten; wobei die Arylgruppe gegebenenfalls durch einen oder mehrere Substitutenten substituiert ist, die aus einem Halogenatom, einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkyloxy-, C₃-C₇-Cycloalkyl-C₁-C₃-alkylenoxy-, C₁-C₆-Fluoralkoxy-, Nitro- oder Cyanogruppe ausgewählt sind;
oder R₆ und R₇ gemeinsam ein Lactam mit 4 bis 7 Ringgliedern, das das Stickstoffatom und die C(O)-Gruppe, von der sie betragen werden, umfasst, bildern.

4. Arzneimittel, **dadurch gekennzeichnet, dass** es eine Verbindung der Formel (I) nach Anspruch 1 oder ein Additionssalz dieser Verbindung mit einer pharmazeutisch unbedenklichen Säure oder ein Hydrat oder ein Solvat der Verbindung Formel (I) umfasst.

5. Pharmazeutische Zusammensetzung, **dadurch gekennzeichnet, dass** sie eine Verbindung der Formel (I) nach Anspruch 1 oder ein pharmazeutisch unbedenkliches Salz, ein Hydrat oder ein Solvat dieser Zusammensetzung sowie mindestens einen pharmazeutisch unbedenklichen Träger umfasst.

6. Verwendung einer Verbindung der Formel (I) nach Anspruch 1 zur Herstellung eines Arzneimitteln zur Vorbeugung und Behandlung von Pathologien, an denen Rezeptoren des TRPV1-Typs impliziert sind, wobei diese Pathologien aus der Reihe Schmerz, Entzündung, Stoffwechselstörungen, Störungen der Harnwege, gynäkologische Störungen, Störungen des Magen-Darm-Trakts, Störungen der Atemwege, Schuppenflechte, Juckreiz, Haut-, Augen- oder Schleimhautreizungen, Herpes, Gürtelrose, Depression und Erkrankungen des Zentralnervensystems ausgewählt sind.

## Claims

1. Compound of the formula (I) 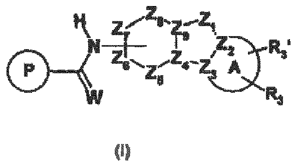 in which:
the group 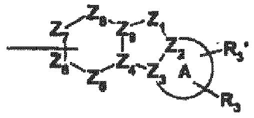 is selected from: 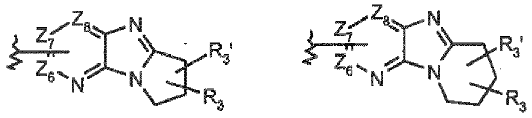
Z₆, Z₇ and Z₈ represent, independently of one another, a carbon atom bonded to the nitrogen atom of the amide of formula (I), or a group C-R₂;
R₂ represents a hydrogen atom;
R₃ and R₃' are carried by a carbon atom and represent, independently of one another, a hydrogen atom, a halogen atom or a C₁-C₆-alkyl, hydroxyl, C₁-C₆-alkyl-O(O)-O-, NR₄R₅, NR₆C(O)R₇ or aryl group;
R₄ and R₅ represent, independently of one another, a hydrogen atom or a C₁-C₆-alkyl group;
R₆ and R₇ represent, independently of one another, a hydrogen atom or a C₁-C₆-alkyl group;
W represents an oxygen atom;
P represents an indolyl or pyrrolo[2,3-b]pyridinyl group as shown below, bonded in position C-2 to the carbonyl of the amide of formula (I): 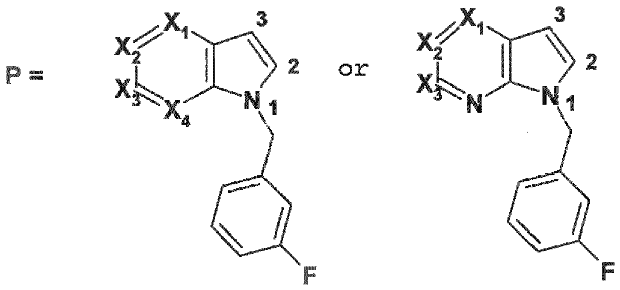
X₁, X₂, X₃ and X₄ represent, independently of one another, a group C-R₁;
R₁ is selected from a hydrogen atom, a halogen atom, C₁-C₆-alkyl, C₁-C₈-fluoroalkyl, C₁-C₆-thioalkyl and C₁-C₆-alkyl-S(O)₂-;
the nitrogen atom or atoms may optionally be in oxidized form (N-oxide)
in the form of a base or addition salt with an acid, and in the form of a hydrate or solvate.

2. Process for preparing a compound of formula (I) according to Claim 1, **characterized in that** a compound of general formula (II): 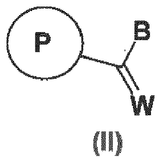 in which P and W are as defined in the general formula (I) according to Claim 1
is reacted with a compound of general formula (III), 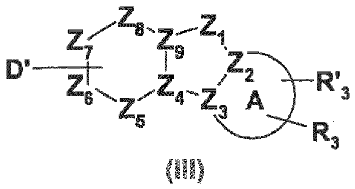 in which A, Z₁, Z₂, Z₃, Z₄, Z₅, Z₆, Z₇, Z₈, Z₆, R₃ and R₃' are as defined in the general formula (I) according to Claim 1;
- when B represents a C₁-C₆-alkoxy, C₃-C₇-cycloalkyloxy, C₃-C₇-cycloalkyl-C₁-C₃-alkylenoxy or aryl-C₁-C₃-alkylenoxy group, with an amide of the compound of general formula (III), under reflux of a solvent, the aluminium amide of the compound of general formule (III) being prepared by prior redaction of trimethylaluminium with the compound of general formula (III) in which D' represents an NH₂ group;
- when B represents a hydroxy group and W represents an oxygen atom,
either by converting the carboxylic acid function into acid halide and then by redacting the compound of general formula (II) in which B represents a chlorine atom and W represents an oxygen atom with the compound of general formula (III) in which D' represents an NH₂ group, in the presence of a base;
or by coupling the compound of general formula (II) with the compounds of general formula (III) in which D' represents an NH₂ group, in the presence of a coupling agent and a base in a solvent;
- when B represents an NH₂ group and W represents an oxygen atom, by reacting the compound of general formula (II) with the compound of general formula (III) in which D' corresponds to a heaving group, in the presence of a copper salt in catalytic amount, in the presence of a catalytic amount of a ligand of the copper, all in the presence of a base in a solvent.

3. Compound of formula (IV) 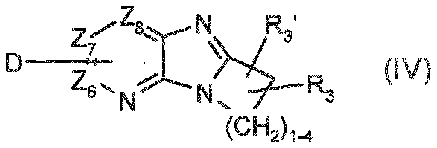 in which Z₆, Z₇ and Z₈ represent, independently of one another, a carbon atom or a group C-R₂; one of Z₆**,** Z₇ and Z₈ corresponds to a carbon atom and carries the group D; D represents a halogen atom or an NH₂ group;
R₂ represents a hydrogen atom;
R₃ and R₃' represent, independently of one another, a hydrogen atom, a halogen atom, a C₁-C₆-alkyl, C₃-C₇-cycloalkyl, C₃-C₇-cycloalkyl-C₁-C₃-alkylene, C₁-C₆-fluoroalkyl, hydroxyl, thiol, C₁-C₆-alkoxy, C₃-C₇-cycloalkyloxy, C₃-C₇-cycloalkyl-C₁-C₃-alkylenoxy, aryl-C(O)-O-, C₁-C₆-alkyl-C(O)-O-, C₃-C₇-cycloalkyl-C(O)-O-, C₃-C₇-cycloalkyl-C₁-C₃-alkylene-C(O)-O, C₁-C₆-fluoroalkyl-C(O)-O-, oxo, thio, NR₄R₅, NR₆C(O)R₇, heteroaryl or aryl group, the heteroaryl and aryl groups being optionally substituted by one or more substituents selected from a halogen atom or a C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro or cyano group;
or else R₃ and R₃', together with the carbon atom which carries them, form a C₃-C₇-cycloalk-1,1-diyl group;
R₄ and R₅ represent, independently of one another, a hydrogen atom or a C₁-C₆-alkyl, C₃-C₇-cycloalkyl, C₃-C₇-cycloalkyl-C₁-C₃-alkylene, aryl-C₁-C₅-alkylene or aryl group, or R₄ and R₅, together with the nitrogen atom which carries them, form an azetidine, pyrrolidine, piperidine, azepine, morpholine, thiomorpholine, piperazine or homopiperazine group, the group NR₄R₅ being optionally substituted by a C₁-C₆-alkyl, C₃-C₇-cycloalkyl, C₃-C₇-cycloalkyl-C₁-C₃-alkylene, aryl-C₁-C₆-alkylene, aryl, heteroaryl, aryl-S(O)₂-, C₁-C₆-alkyl-S(O)₂-, C₁-C₆-fluoroalkyl-S(O)₂, C₃-C₇-cycloalkyl-S(O)₂-, C₃-C₇-cycloalkyl-C₁-C₃-alkylene-S(O)₂-, aryl-C(O)-, C₁-C₆-alkyl-C(O)-, C₃-C₇-cycloalkyl-C(O)-, C₃-C₇-cycloalkyl-C₁-C₃-alkylene-C(O)-, C₁-C₆-fluoroalkyl-C(O)-, hydroxy, C₁-C₆-alkyloxy, C₃-C₇-cycloalkyloxy, C₃-C₇-cycloalkyl-C₁-C₃-alkylenoxy, C₁-C₆-fluoroalkyl, aryloxy-C₁-C₆-alkylene, aryloxy, heteroaryloxy-C₁-C₆-alkylene or heteroaryloxy group;
R₆ and R₇ represent, independently of one another, a hydrogen atom, a C₁-C₆-alkyl, C₃-C₇-cycloalkyl, C₃-C₇-cycloalkyl-C₁-C₃-alkylene, aryl-C₁-C₆-alkylene or aryl group, the aryl group being optionally substituted by one or more substituents selected from a halogen atom, a C₁-C₆-alkyl, C₃-C₇-cycloalkyl, C₃-C₇-cycloalkyl-C₁-C₃-alkylene, C₁-C₆-fluoroalkyl, C₁-C₆-alkoxy, C₃-C₇-cycloalkyloxy, C₃-C₇-cycloalkyl-C₁-C₃-alkylenoxy, C₁-C₆-fluoroalkoxy, nitro or cyano group;
or R₆ and R₇ together form a lactam of 4 to 7 members comprising the nitrogen atom and the group C(O) which carry them.

4. Medicament **characterized in that** it comprises a compound of formula (I) according to Claim 1, or an addition salt of this compound with a pharmaceutically acceptable acid, or else a hydrate or solvate of the compound of formula (I).

5. Pharmaceutical composition **characterized in that** it comprises a compound of formula (I) according to Claim 1, or a pharmaceutically acceptable salt, a hydrate or a solvate of this compound, and also at least one pharmaceutically acceptable excipient.

6. Use of a compound of formula (I) according to Claim 1 for preparing a medicament intended for prevention and treatment of pathologies involving TRPV1 receptors, the said pathologies being selected from pain, inflammation, metabolic disorders, urological disorders, gynaecological disorders, gastrointestinal disorders, respiratory disorders, psoriasis, pruritis, dermal, ocular or mucosal irritation, herpes, shingles, repression and diseases of the central nervous system.
